# EUROPÄISCHE PATENTSCHRIFT

(11) **EP 3 377 902 B1**
(45) Veröffentlichungstag und Bekanntmachung des Hinweises auf die Patenterteilung: **16.09.2020**
(21) Anmeldenummer: 16798628.0
(22) Anmeldetag: 18.11.2016
(51) Int. Cl.: G01N 33/569, G01N 21/00, B01L 3/00

(54) **VERFAHREN UND VORRICHTUNG ZUM NACHWEIS VON BAKTERIEN**
METHOD AND DEVICE FOR DETECTING BACTERIA
PROCEDE ET DISPOSITIF DE CONTROLE DE BACTERIES

(30) Priorität: 20.11.2015 EP 15003307
(43) Veröffentlichungstag der Anmeldung: 26.09.2018
(73) Patentinhaber: Sinamira AG, 82031 Grünwald (DE); Friedrich-Alexander-Universität Erlangen-Nürnberg, 91054 Erlangen (DE)
(72) Erfinder: MIEDL, Walter, 9050 Appenzell (CH); FRIEDRICH, Oliver, 90408 Nürnberg (DE); GILBERT, Daniel, 90482 Nürnberg (DE)
(74) Vertreter: Kador & Partner PartG mbB
(86) Internationale Anmeldenummer: PCT/EP2016/001925
(87) Internationale Veröffentlichungsnummer: WO 2017/084754

(56) Entgegenhaltungen:
- EP-A1- 2 282 204
- EP-A1- 2 905 607
- WO-A1-2006/105504
- WO-A1-2013/096801
- US-A1- 2001 006 783
- P.A. MOSIER-BOSS ET AL.: "Use of Fluorescently Labeled Phage in the Detection and Identification of Bacterial Species", APPLIED SPECTROSCOPY, Bd. 57, 2003, Seiten 1138-1144, XP002754992, in der Anmeldung erwähnt
- "VWR Collection filtration VWR Collection products: delivering high quality & superior performance A collection of filtration products and speciality papers", , 1. Januar 2010 (2010-01-01), XP055253122, Gefunden im Internet: URL:https://si.vwr.com/store/asset?assetUR I=https://si.vwr.com/stibo/hi_res/std.lang .all/52/55/11935255.pdf [gefunden am 2016-02-25]

## Beschreibung

Die vorliegende Erfindung betrifft ein Verfahren zum Nachweis von Bakterien, insbesondere ein beschleunigtes und hoch-sensitives Verfahren zum Nachweis von bakteriellen Erregern, welches auf der Bildung von spezifischen Bakterien-Bakteriophagen-Komplexen beruht. Darüber hinaus werden ein Reaktionsgefäß sowie die Verwendung dieses Reaktionsgefäßes und eines Messgeräts für die Durchführung des erfindungsgemäßen Verfahrens offenbart.

Bakterien zeichnen sich oftmals als Erreger ernstzunehmender Erkrankungen aus. Insbesondere sind multiresistente Bakterienstämme, welche auch als "Krankenhaus-Keime" bezeichnet werden, für eine Vielzahl von bakteriellen Infektionen in Krankenhäusern verantwortlich. Gerade angesichts des demographischen Wandels und der Möglichkeiten der modernen Intensivmedizin, finden sich auf einer Intensivstation zunehmend ältere Patienten, welche besonders anfällig für Infektionen durch Bakterien sind. Einer der zentralen Bausteine intensivmedizinischer Therapie bildet die mikrobiologische Diagnostik. Eine schnelle und korrekte Identifizierung der Erreger einer Infektion beeinflusst in erheblicher Weise nicht nur die Letalität der Patienten, sondern ermöglicht auch den keimspezifischen Antibiotikaeinsatz, so dass eine inflationäre Verwendung von Breitbandantibiotika und die damit verbundene Selektion multiresistenter Bakterienstämme vermieden werden können. Allerdings sind die bisherigen Verfahren meist zeitaufwendig und ebenso kostenintensiv.

So bildet die Blutkultur bisher einen der Hauptpfeiler in der Diagnosestellung von Blutstrominfektionen. Hierbei findet die Bebrütung in Kulturflaschen unter aeroben und anaeroben Bedingungen statt. Geeignet sind dazu neben Blut sterile Flüssigkeiten wie Punktate oder Liquorentnahmen. Im Allgemeinen werden etwa 3-4 Kulturflaschen mit jeweils 10 ml Vollblut zur Diagnostik bei Raumtemperatur entnommen. Bebrütet werden die Blutkulturflaschen in einem halbautomatischen System, bei denen das zunehmende Wachstum von Bakterien an der zunehmenden CO₂-Produktion oder einer Flaschendruckzunahme gemessen wird. Die Routinebebrütung beträgt in den meisten Fällen 48-72 Stunden. Besondere Probleme bereiten dabei langsam wachsende Bakterien, die eine Bebrütung von 5 bis zu 21 Tagen erfordern können und somit leicht in der Routine übersehen werden. Aber auch spezielle Nährmedien oder Färbeverfahren verkomplizieren und verzögern eine ausreichende und schnelle Diagnosestellung. Ist eine Blutkulturflasche positiv detektiert, wird diese aus dem System selektiert. Aus dieser Flasche wird eine Probe entnommen und auf verschiedenen Nährmedien werden wiederum aerobe und anaerobe Subkulturen angefertigt. Nach etwa acht weiteren Stunden kann eine grobe Resistenzbestimmung bei begleitender Antibiotikatestung erfolgen.

Eine schnellere Erregeridentifizierung gelingt direkt aus der positiven Blutkulturflasche innerhalb von 1-2 Stunden mittels Latexagglutination zum Nachweis von Antigenen von *Streptococcus pneumoniae, Streptococcus agalactiae, Haemophilus influenzae, Neisseria meningitis, E. coli* und *Salmonella typhi.* Nukleinsäurenachweisverfahren, wie die DNA-Hybridisierung, Fluoreszenz-in-situ-Hybridisierung (FISH) und spezifische Nukleinsäureamplifikationstechniken (NAT) werden zur Bakteriämie- und Sepsisdiagnostik in der jüngeren Zeit eingesetzt, stehen aber nur in sehr wenigen Laboren zur Verfügung. Die Erregerbestimmung gelingt dabei innerhalb von 1-3 Stunden aus einer positiven Blutkulturflasche. Daneben sind in den letzten Jahren einige klinische Labore mit MALDI-TOF (matrixunterstützte Laserdesorption/ Ionisation-Time of Flight)-Massenspektrometern erweitert worden, welche eine Bakterienidentifizierung innerhalb nur weniger Minuten erlauben. Allerdings erfordern auch die oben beschriebenen modernen Nachweismethoden eine positiv detektierte Blutkulturflasche. Daher kann mittels einer positiven Blutkultur der Nachweis von bakteriellen Erregern in der Regel erst innerhalb eines Zeitfensters von 24 bis 36 Stunden nach Entnahme der Probe gelingen.

Zum Teil ist es durch PCR-diagnostische Verfahren möglich, schon innerhalb von 4-6 Stunden nach der Entnahme Bakteriennachweise aus Blutproben ohne den Zwischenschritt der Bebrütung einer Blutkultur zu erhalten, z.B. für *Staphylococcus aureus.* Die modernsten Verfahren sind die Multiplex-Amplifikationsverfahren, welche die häufigsten Erregerarten erfassen können. Dazu gehören beispielsweise LightCycler, SeptiFast und SepsiTest. Allerdings sind diese Untersuchungen recht anfällig für Kontaminationen, was falsche oder falsch positive Ergebnisse liefert. Nachteilig zeigen sich bei diesen Verfahren zudem der Arbeitsaufwand und die Kostenstruktur, da für die PCR-Diagnostik neben dem Material, entsprechendes Fachpersonal sowie eine geeignete Infrastruktur benötigt werden.

Darüber hinaus ist zu beachten, dass in 2-3 % der Fälle eine positiv detektierte Blutkultur ebenfalls auf eine Kontamination der Probe oder Verarbeitungsfehler nach der Probenentnahme zurückgeht. Die Folge sind fehlerhafte antibiotische Therapien mit unnötigen und zum Teil erheblichen Kosten, weshalb ein schnelles, einfach zu handhabendes und hoch-sensitives Verfahren zum Nachweis der bakteriellen Erreger wünschenswert wäre.

Bezüglich der Erfordernisse einer hohen Sensitivität und Spezifität bieten Bakteriophagen (kurz Phagen) einen vielversprechenden Ansatzpunkt. Bakteriophagen sind Viren, die sich spezifisch nur gegen Bakterienzellen richten, diese über Oberflächen-Rezeptorstrukturen erkennen, daran Schlüssel-Schloss-ähnlich binden und in den nachfolgenden Schritten die genetische und enzymatische Ausstattung der Bakterien für ihre eigene Vermehrung nutzen. Die befallenen Bakterienzellen lysieren und entlassen zahlreiche neue Phagen. Diese biologische Prozesskette geht so lange weiter, bis alle für die freien Phagen erreichbaren Wirtsbakterienzellen entfernt sind. Phagen befallen fast immer nur Stämme innerhalb einer Bakterienart, d.h. sie sind jeweils hoch-spezifisch für eine bestimmte Bakterienart. Da viele Bakterienarten ubiquitär in zahlreichen Habitaten vorkommen, können gleich einem biologischen Prinzip dort auch ihre komplementären Phagen vermutet werden. Global übertrifft die von Experten geschätzte Zahl der Phagen die der Bakterien um das Zehnfache (C. Rohde & J. Sikorski: Bakteriophagen - Vielfalt, Anwendung und ihre Bedeutung für die Wissenschaft vom Leben. Naturwiss. Rundschau (2011), 64, 5-14). Die gewünschten Bakteriophagen können oft aus allen denkbaren wässrigen Habitaten durch einfache Beprobung und nachfolgendes Screening gegen die Zielbakterien gewonnen werden.

Aufgrund der enormen Wirtsspezifität und der lytischen Eigenschaften werden Phagen in der klinischen Forschung und Analytik bereits vielfältig verwendet, z.B. in der Phagentherapie gegen bakterielle Infektionen. Ferner beschreiben Thanki, Malik, Clokie die Entwicklung eines biolumineszenten Reporter-Phagen zur Identifizierung von *Clostridium difficile,* bei dem die Gen-Kassetten *luxA* und *LuxB,* welche das Enzym Luziferase codieren, in ein Plasmid kloniert werden zur Konjugation in einen *Clostridium difficile*-spezifischen Phagen und zur homologen Insertion in das Phagengenom (A. Thanki, D. Malik, M. Clokie, The Development of a Reporter Phage for the Identification of Clostridium difficile, Bacteriophages (2015), Abstract, 25). Die wissenschaftliche Publikation von Mosier-Boss et al. beschreibt weiterhin den spezifischen Nachweis von *Salmonella typhimurium LT2* unter Verwendung fluoreszenzmarkierter P22-Phagen. Die Markierung der Phagen ist bei dem beschriebenen Ansatz komplett auf die Phagen DNA beschränkt (10 min Inkubation von P22 mit SYBR gold), welche bei Infektion in das Wirtsbakterium injiziert wird. Die Detektion spezifischer *Salmonella typhimurium-P22-Phagen-*Komplexe erfolgt nach deren Isolierung mittels Fluoreszenz-Mikroskopie. (P.A. Mosier-Boss et al., Use of Fluorescently Labeled Phage in the Detection and Identification of Bacterial Species, Applied Spectroscopy (2003), 57, 1138-1144).

Auch in der Lebensmittelindustrie und bei der Trinkwasserversorgung ist ein einfacher und sicherer Bakteriennachweis wichtig. Im Trinkwasser können sich Legionella-Stämme bei Temperaturen zwischen 20 und 45 °C vermehren, sodass eine regelmäßige Untersuchung des Trinkwassers geboten und auch in vielen Ländern gesetzlich vorgeschrieben ist. Die deutsche Trinkwasserverordnung schreibt beispielsweise eine regelmäßige Untersuchungspflicht auf Legionellen vor. Es ist zu erwarten, dass die gesetzlichen Bestimmungen weiter verschärft werden. Institute und Behörden, wie z.B. Trinkwasserlabore oder Gesundheitsämter sind demnach bestrebt, einen schnellen, einfachen, sensitiven und sicheren Bakteriennachweis auf Legionella-Stämme durchzuführen. Hieran fehlt es derzeit, und eine Verbesserung ist wünschenswert.

Andererseits sind durch Lebensmittel übertragene bakterielle Lebensmittelintoxikationen und Lebensmittel-Toxin-Infektionen ein weltweites Problem und verursachen massive wirtschaftliche Schäden. Die lange Zeitspanne bis zum sicheren Nachweis der Erreger bedeutet einen erheblichen ökonomischen und wirtschaftlichen Verlust, da sich entweder die Lagerzeit der Produkte bis zum Weiterverkauf verlängert oder es sogar zum Rückruf des entsprechenden Lebensmittels kommen kann. Eine schnellere und sichere Nachweismethode kann hier einen wesentlichen Anteil zur Lebensmittelsicherheit und der Minimierung wirtschaftlicher Verluste beitragen. Gefürchtete Erreger im Lebensmittelbereich sind u.a. *Cronobacter spp., Salmonella spp., Shigella spp., Escherichia coli, Yersinia enterocolitica, Vibrio spp., Aeromonas spp., Plesiomonas spp., Listeria monocytogenes, Campylobacter jejuni,* oder *Bacillus cereus.* Ein schneller Ausschluss oder Nachweis von Erregern in Lebensmitteln ist daher für deren Freigabe essenziell.

Weitere Gebiete, in denen schnelle und einfache Bakteriennachweise wünschenswert sind, beinhalten die Agrarindustrie, die Veterinärmedizin oder Anwendungen im militärischen Bereich oder Katastrophenschutz.

Die Veröffentlichung von P.A. Mosier-Boss et al. ("Use of Fluorescently Labeled Phage in the Detection and Identification of Bacterial Species", Applied Sectroscopy 2003, 57, 1138-1144) beschreibt den spezifischen Nachweis von *Salmonella typhimurium LT2* Bakterien mit Hilfe fluoreszenzmarkierter SYBR Gold P22 Bakteriophagen. Dabei werden die Bakterien mit den gelabelten Phagen versetzt, inkubiert und die Suspension schließlich durch einen Whatman-Filter der Porengröße 0,2 µm filtriert. Der Überstand auf dem Filter, d.h. die entstandenen Bakterien-Bakteriophagen-Komplexe, werden anschließend unter dem Fluoreszenzmikroskop analysiert.

Weiterhin offenbart WO 2006/105504 A1 ein Verfahren sowie eine Vorrichtung zum Nachweis von Mikroorganismen. Dabei umfasst die Vorrichtung markierte Bakteriophagen sowie ein Gefäß mit zwei Kammern und einem Trennelement, wobei das Trennelement die beiden Kammern voneinander trennt. Ferner kann das Trennelement ein Filter (Porengröße 0.2 µm) oder eine Membran sein, welches die markierten Bakteriophagen passieren lässt während es die Mikroorganismen zurückhält. Zudem können neben Bakterien-Bakteriophagen-Komplexen der einen Trennfraktion auch ungebundene Bakteriophagen der anderen Trennfraktion detektiert werden.

Darüber hinaus werden in US 2001/0006783 A1 ein Verfahren sowie eine Vorrichtung zum Nachweis von Bakterien beschrieben, wobei der Nachweis auf der Messung der Fluoreszenz von gelabelter Phagen-DNS nach Injektion in die Bakterien beruht. Dabei wird ein Bakterien-Detektor, welcher ein optisches Detektionsmittel enthält, wie z.B. ein (Fluoreszenz)-Mikroskop, ein (Fluoreszenz)-Spektrophotometer oder ein Fluoreszenz-Detektor, verwendet. Zudem können die Vorrichtungen einen Bildprozessor zur prozessorgestützten Auswertung der Detektions-Signale enthalten.

Die WO 2013/096801 A1 offenbart ferner Vorrichtungen und Verfahren zum Durchführen optischer und elektrochemischer Assays insbesondere Vorrichtungen mit optischen und elektrochemischen Detektoren und Verfahren zum Durchführen optischer und elektrochemischer Assays unter Verwendung solcher Vorrichtungen. Diese sind besonders für die patientennahe Diagnostik geeignet.

Im Hinblick auf die Nachteile der klinischen Nachweisverfahren für Bakterien aus dem Stand der Technik, insbesondere bezüglich Zeitaufwand, Komplexität und Kostenintensität, bedarf es einer neuen schnelleren und spezifischeren Diagnostik für einen zeitnahen und selektiven Bakteriennachweis, sei es für klinische, human- oder veterinärmedizinische, agrarindustrielle oder militärische Anwendungen, oder für die Trinkwasseranalytik, um nur einige spezifische Anwendungen zu nennen. Daher besteht die Aufgabe der vorliegenden Erfindung darin, ein verbessertes Verfahren zum Nachweis von Bakterien bereitzustellen, welches eine schnellere und verlässliche Identifikation von bakteriellen Erregern, eine vereinfachte Handhabung und eine hohe Sensitivität bei geringeren Kosten liefert. Ebenso soll ein Reaktionsgefäß für die Durchführung des erfindungsgemäßen Verfahrens bereitgestellt werden.

Die erfinderische Lösung der oben genannten Aufgabe basiert auf der evolutionär konservierten, hoch-spezifischen Wechselwirkung zwischen Bakterien und Bakteriophagen.

So offenbart die vorliegende Erfindung ein Verfahren zum Nachweis von Bakterien, umfassend die Schritte
A) Bereitstellen einer oder mehrerer Suspensionen, jeweils umfassend mindestens eine Spezies von markierten Testbakteriophagen, welche spezifisch an eine nachzuweisende Bakterienart bindet;
B) Zugabe einer Probe, die auf das Vorliegen mindestens einer nachzuweisenden Bakterienart getestet werden soll, zu der einen oder den mehreren Suspensionen;
C) Filtration des resultierenden Reaktionsgemisches, wobei ein Filter mit einer Porengröße von 0,1 µm bis 0,5 µm verwendet wird;
D) Detektion von Bakterien-Bakteriophagen-Komplexen im Retentat, bei Vorliegen mindestens einer nachzuweisenden Bakterienart, wobei die Komplexe aus Bakterien der mindestens einen nachzuweisenden Bakterienart und daran gebundenen Testbakteriophagen der mindestens einen Spezies von Testbakteriophagen bestehen;
E) Detektion von ungebundenen Testbakteriophagen im Filtrat;
F) prozessorgestützte Verarbeitung von erhaltenen Signalen, welche durch die Detektion in den Schritten D) und E) erzeugt werden und Ausgabe von Detektionsergebnissen an einen Benutzer;
wobei die eine oder mehreren Suspensionen ferner einen markierten Referenz-Bakteriophagen umfassen, welcher an keine nachzuweisende Bakterienart bindet und eine von den Testbakteriophagen disjunkte Markierung aufweist, so dass der markierte Referenz-Bakteriophage ebenfalls im Schritt E) detektiert wird; oder wobei, alternativ zu einem markierten Referenz-Bakteriophagen, markierte Mikro- oder Nanopartikel verwendet werden, welche an keine nachzuweisende Bakterienart binden und den Filter aufgrund ihrer geringen Größe passieren können.

Ferner betrifft die vorliegende Erfindung ein Reaktionsgefäß zur Durchführung des erfindungsgemäßen Verfahrens zum Nachweis von Bakterien, umfassend mindestens zwei Kompartimente, welche durch einen Filter voneinander getrennt sind, wobei der Filter eine Porengröße von 0,1 µm bis 0,5 µm aufweist, wobei ein Kompartiment ein Phagenreservoir ist, welches eine Suspension enthält, die mindestens eine Spezies von markierten Testbakteriophagen umfasst, welche spezifisch an eine nachzuweisende Bakterienart bindet; und ein Kompartiment ein Auffangreservoir ist, zur Aufnahme des Filtrats nach Zugabe einer Probe zu der Suspension und Filtration durch den Filter;
ein Mittel zur Identifikation des Reaktionsgefäßes; und
mindestens zwei Messfenster, wobei ein Messfenster im Bereich der Filteroberfläche, welche dem Phagenreservoir zugewandt ist, angeordnet ist, und ein Messfenster im Bereich des Auffangreservoirs angeordnet ist,
wobei die Suspension ferner einen markierten Referenz-Bakteriophagen umfasst, welcher an keine nachzuweisende Bakterienart bindet und eine von den Testbakteriophagen disjunkte Markierung aufweist; oder wobei das Reaktionsgefäß ein weiteres Kompartiment zwischen Filter und Phagenreservoir umfasst, welches eine Indikatorlösung mit markierten Mikro- oder Nanopartikeln enthält, welche nicht an eine nachzuweisende Bakterienart binden und den Filter aufgrund ihrer geringen Größe passieren können.

Darüber hinaus betrifft die vorliegende Erfindung eine Kartusche, die zwei oder mehrere erfindungsgemäße Reaktionsgefäße umfasst, welche parallel und/oder seriell zueinander angeordnet sind,
wobei jedes parallele Reaktionsgefäß unterschiedliche Spezies von markierten Testbakteriophagen in der Suspension des Phagenreservoirs enthält, welche jeweils spezifisch an unterschiedliche nachzuweisende Bakterienarten binden.

Zusätzlich bezieht sich die vorliegende Erfindung auf die Verwendung eines Messgeräts zur Durchführung des erfindungsgemäßen Verfahrens zum Nachweis von Bakterien, wobei das Messgerät umfasst:
einen Schacht, in den ein erfindungsgemäßes Reaktionsgefäß oder eine erfindungsgemäße Kartusche eingeschoben wird;
mindestens zwei Detektions-Optiken, welche jeweils im Bereich der Messfenster des eingeschobenen Reaktionsgefäßes oder der Kartusche angeordnet sind, wobei die Detektions-Optiken jeweils ein Linsensystem zur Fokussierung von emittiertem Licht der Markierung der Bakteriophagen auf mindestens einen Sensor umfassen, wobei der mindestens eine Sensor das emittierte Licht detektiert;
einen Prozessor, welcher jeweils mit dem mindestens einen Sensor der Detektions-Optiken verbunden ist und welcher die erhaltenen Detektionssignale verarbeitet; und
eine mit dem Prozessor verbundene Ausgabeeinheit, welche die Detektionsergebnissen an einen Benutzer ausgibt.

Die vorteilhafte Wirkung der vorliegenden Erfindung liegt einerseits in der schnellen und hoch-sensitiven Identifizierung von bakteriellen Erregern. So ist für das erfindungsgemäße Verfahren kein Anlegen einer Blutkultur notwendig. Vielmehr kann eine Probe, welche auf das Vorliegen einer bestimmten Bakterienart getestet werden soll, direkt nach der Probenentnahme verwendet werden. Dadurch erfolgt ein Bakteriennachweis im Idealfall, ohne einen notwendigen Transport und Lagerung der Probe, in weniger als 1 Stunde. Dies ermöglicht eine schnellere und keimspezifischere Anwendung von Antibiotika, wodurch eine prophylaktische Verwendung von Breitbandantibiotika vermieden wird. Zudem können mit Hilfe des erfindungsgemäßen Verfahrens Bakterien bereits ab einer Konzentration von weniger als 100 Bakterien pro Milliliter nachgewiesen werden. Andererseits zeichnet sich das vorliegende Verfahren durch die unmittelbare Verwendung der Messprobe nach deren Entnahme und Einfachheit in der Handhabung aus, so dass es weniger anfällig für Verarbeitungsfehler ist. Darüber hinaus erfordert die Durchführung des Verfahrens mit Hilfe des erfindungsgemäßen Reaktionsgefäßes und des erfindungsgemäßen Messgeräts kein ausgewiesenes Fachpersonal oder eine spezielle Infrastruktur, was den Kostenaufwand erheblich reduziert.

### Figuren

- Figur 1:: Schematische Darstellung einer bevorzugten Ausführungsform des erfindungsgemäßen Verfahrens zum Nachweis von Bakterien
- Figur 2:: Schematische Darstellung einer weiteren bevorzugten Ausführungsform des erfindungsgemäßen Verfahrens zum Nachweis von Bakterien
- Figur 3 A:: Schematische Darstellung einer bevorzugten Ausführungsform des erfindungsgemäßen Reaktionsgefäßes zum Nachweis von Bakterien
- Figur 3 B:: Theoretischer Verlauf einer zeitaufgelösten Messung der Fluoreszenz in einer bevorzugten Ausführungsform des erfindungsgemäßen Verfahrens unter Verwendung des erfindungsgemäßen Reaktionsgefäßes aus Figur 3 A.
- Figur 4 A:: Schematische Darstellung einer bevorzugten Ausführungsform der erfindungsgemäßen Kartusche zum Nachweis von Bakterien in a) Vorderansicht, b) Aufsicht und c) Seitenansicht.
- Figur 4 B: Theoretische Verläufe von zeitaufgelösten Messungen der Fluoreszenz in einer bevorzugten Ausführungsform des erfindungsgemäßen Verfahrens unter Verwendung der erfindungsgemäßen Kartusche aus Figur 4 A.
- Figur 5:: Schematische Darstellung des Aufbaus von erfindungsgemäßem Reaktionsgefäß und Messgerät in einer Ausführungsform.
- Figur 6:: RFID Chip als Mittel zur Identifizierung des erfindungsgemäßen Reaktionsgefäßes.
- Figur 7:: Markierung eines TB54-Bakteriophagen mit Fluorescein-NHS-Estern.
- Figur 8:: Anregungs- und Emissionsspektrum von Fluorescein-markierten TB54 Bakteriophagen.
- Figur 9 A:: Fluoreszenzmikroskopische Aufnahme der Bakterien-Bakteriophagen-Interaktion.
- Figur 9 B:: Hellfeldaufnahme der Bakterien-Bakteriophagen-Interaktion.
- Figur 10 A:: Optische Schnittbilder von mit Fluorescein-markierten TB54 Bakteriophagen infizierten *Escherichia coli* Bakterien im Durchlicht und in fluoreszenzmikroskopischer Darstellung.
- Figur 10 B:: Dreidimensionale Rekonstruktionen von mit Fluoresceinmarkierten TB54 Bakteriophagen infizierten *Escherichia coli* Bakterien.
- Figur 11 A:: Fluoreszenzspektren von Fluorescein-markierten TB54 Bakteriophagen bei unterschiedlichen Bakteriophagenkonzentrationen.
- Figur 11 B:: Darstellung der Integrale der zwischen 520 und 530 nm der Fluoreszenzspektren aus Figur 10 A bei unterschiedlichen Bakteriophagenkonzentrationen.
- Figur 12 A-C:: Messung der optischen Dichte einer seriell verdünnten Bakteriensuspension, Darstellung der detektierten Fluoreszenzsignale von E. *coli* C600-TB54-Phagen-Komplexen auf der Filteroberfläche im Retentat sowie Darstellung der detektierten Fluoreszenzsignale ungebundener TB54-Phagen im Filtrat, jeweils bei verschiedenen Bakterienkonzentrationen.

Das erfindungsgemäße Verfahren umfasst im Schritt A) die Bereitstellung einer oder mehrerer Suspensionen, welche jeweils mindestens eine Spezies von markierten Testbakteriophagen umfasst, welche spezifisch an eine nachzuweisende Bakterienart bindet. Vorzugsweise ist die Markierung für die jeweilige Spezies von markierten Testbakteriophagen kennzeichnend. Dies bedeutet, die Markierungen, die an unterschiedliche Spezies von Testbakteriophagen gekoppelt sind, sind jeweils disjunkt. Alternativ können die Markierungen auch teilweise überlappend eingestellt werden, wobei die Signale der einzelnen Markierungen bzw. der unterschiedlichen Spezies von Testbakteriophagen im nachgeschalteten Prozessionsverfahren mittels eines spektroskopischen Analyseverfahrens spektral entmischt und damit eindeutig zugeordnet werden können. In diesem Fall kann die Detektion überlappender Markierungen ebenfalls sequentiell erfolgen.

Vorzugsweise beträgt das Volumen der Suspension zwischen 0,5 und 5,0 ml.

Die Konzentration der Bakteriophagen in der Suspension, auch als Phagentiter bezeichnet, beträgt vorzugsweise 10³-10¹² pfu/ml (pfu = "plaque forming unit"), weiter vorzugsweise 10⁴-10¹⁰ pfu/ml. Am meisten bevorzugt ist ein Phagentiter in der Suspension von 10⁵-10⁷ pfu/ml.

Je nachdem welche Bakterienart nachgewiesen werden soll, wird eine Spezies von Testbakteriophagen für die Herstellung der Suspension ausgewählt, welche spezifisch an diese Bakterienart bindet, d.h. spezifisch diese Bakterienart infiziert. Aufgrund der evolutionär konservierten, enormen Wirtsspezifität der Bakteriophagen ist überdies ein spezifischer Nachweis einzelner Stämme innerhalb einer Bakterienart möglich. In diesem Fall wird eine Spezies von Testbakteriophagen für die Herstellung der Suspension ausgewählt, welche spezifisch an einen bestimmten Stamm innerhalb einer Bakterienart bindet. Dabei ist eine Bindung der ausgewählten Spezies von Testbakteriophagen an eine andere als die nachzweisende Bakterienart bzw. an einen anderen als den nachzuweisenden Stamm nahezu ausgeschlossen. Infolgedessen ist die Möglichkeit, einen falsch-positiven Nachweis durch das erfindungsgemäße Verfahren zu erhalten, minimal und das Ergebnis des erfindungsgemäßen Nachweisverfahrens äußerst verlässlich. Bei der Bereitstellung mehrerer Suspensionen können dementsprechend unterschiedliche Spezies von Testbakteriophagen, welche jeweils spezifisch an unterschiedliche nachzuweisende Bakterienarten binden, ausgewählt werden, so dass eine Suspension jeweils eine bestimmte Spezies von Testbakteriophagen enthält. Damit wird ermöglicht, mehrere Bakterienarten in einer Probe nachzuweisen. Um im weiteren Verfahren die Testbakteriophagen der mindestens einen Spezies und die entstehenden Bakterien-Bakteriophagen-Komplexe detektieren zu können, tragen die Testbakteriophagen eine Markierung. Vorzugsweise ist die Markierung für die jeweilige Spezies von Testbakteriophagen kennzeichnend, d.h. alle Testbakteriophagen einer Spezies tragen die gleiche Markierung, welche von den Markierungen anderer Spezies unterscheidbar ist.

Zur Herstellung der Suspension werden die markierten Testbakteriophagen der jeweiligen mindestens einen Spezies vorzugsweise in Wasser oder in einer geeigneten Pufferlösung oder sonstigen wässrigen Lösung suspendiert. Als geeignete Pufferlösungen werden vorzugsweise 0,01-0,3 M Phosphatpuffer, pH 6,5-9,5; 0,01-0,3 M Carbonat-puffer, pH 6,5-9,5; phosphatgepufferte Salzlösung (PBS) oder Mischungen davon verwendet. Weiter vorzugweise enthalten das Wasser und die geeigneten Pufferlösungen jeweils MgCl₂, MgSO₄ und/oder CaCl₂. Die Konzentration von MgCl₂, MgSO₄ und CaCl₂ beträgt vorzugsweise jeweils 1 bis 15 mM.

In Schritt B) des erfindungsgemäßen Verfahrens wird eine Probe, die auf das Vorliegen mindestens einer zu nachzuweisenden Bakterienart getestet werden soll, zu der einen Suspension oder zu den mehreren Suspensionen gegeben. Vorzugsweise besteht die Probe aus einer humanen oder tierischen Körperflüssigkeit, wie Blut, Urin, Speichel oder einer anderen Körperflüssigkeit, einem Abstrich, welcher von einem Menschen oder einem Tier entnommen wird, aus Wasser aus einem Trinkwassersystem oder in wässriger Lösung aufbereitete Lebensmittel oder Lebensmittelbestandteile. Die Probe kann nach ihrer Entnahme direkt, ohne weitere Vorbehandlung zu der Suspension bzw. den Suspensionen gegeben werden. Lebensmittel oder Lebensmittelbestandteile werden in wässrige Lösung gebracht. Dadurch wird der Zeitaufwand für den Bakteriennachweis erheblich reduziert und die Gefahr einer Kontamination durch Transport und/oder Lagerung minimiert. Ferner erlaubt das erfindungsgemäße Verfahren ebenso den Nachweis von Bakterien aus einer Probe, welche aus einer angelegten Bakterienkultur stammt.

Vorzugsweise wird die Probe vor Zugabe durch einen Grobfilter mit einer Porengröße von 0,5-10,0 µm filtriert, um etwaige Verunreinigungen durch Partikel (> 0,5 µm) zu entfernen, damit ein mögliches Verstopfen des Filters im nachfolgenden Schritt C) vermieden werden kann. Alternativ kann auch ein mehrstufiger Grobfilter mit abnehmenden Porengrößen von 0,5-10 µm verwendet werden.

Das Volumen der Probe beträgt vorzugsweise 0,05-2,5 ml, weiter vorzugweise 0,1-2,0 ml und am meisten bevorzugt 0,5-2,0 ml. Es sind auch Kombinationen der genannten Volumenbereiche möglich.

Vorzugsweise wird die Probe in einem Schritt G), welcher auf Schritt B) folgt, mit der Suspension vermischt und das resultierende Reaktionsgemisch inkubiert. Das Mischen erfolgt vorzugsweise durch mehrmaliges Schwenken oder moderates Rühren. Das entstehende Reaktionsgemisch wird vorzugsweise 2-30 min, weiter vorzugweise 5-15 min, noch weiter vorzugsweise 5-10 min inkubiert. Die Temperatur während der Inkubation beträgt vorzugsweise 4-60°C, weiter vorzugsweise 10-30°C. Eine Inkubation des Reaktionsgemisches bei Raumtemperatur (RT, 20-25°C) ist am meisten bevorzugt. Durch das Mischen und die Inkubation soll, bei Vorliegen mindestens einer zu nachzuweisenden Bakterienart, sichergestellt werden, dass jedes nachzuweisende Bakterium vor der nachfolgenden Filtration in Schritt C) von den markierten Testbakteriophagen der jeweiligen mindestens einen Spezies gebunden bzw. infiziert wird.

Vorzugsweise wird für die Filtration des Reaktionsgemisches in Schritt C) ein Filter mit einer Porengröße von 0,1-0,5 µm verwendet. Weitervorzugsweise beträgt die Porengröße des verwendeten Filters 0,2-0,5 µm, besonders bevorzugt ist eine Porengröße von 0,3-0,5 µm und am meisten bevorzugt ist eine Porengröße von 0,2-0,4 µm. Die Porengröße des verwendeten Filters wird so gewählt, dass die mindestens eine nachzuweisende Bakterienart sowie Bakterien-Bakteriophagen-Komplexe, welche aus Bakterien der mindestens einen nachzuweisenden Bakterienart und daran gebundenen Testbakteriophagen der mindestens einen Spezies bestehen, zurückgehalten und auf der Filteroberfläche immobilisiert werden, während ungebundene Bakteriophagen den Filter passieren können. Dadurch wird eine Trennung von Bakterien-Bakteriophagen-Komplexen der nachzuweisenden Bakterienart und sonstigen eventuell vorliegenden Bakterien von ungebundenen Bakteriophagen erreicht. Die Porengröße des verwendeten Filters wird somit entsprechend der Größe und Morphologie der jeweiligen nachzuweisenden Bakterienart gewählt.

Der Filter besteht vorzugsweise aus einer Membran, einer Folie oder einer Matrix, wie einem Kugelbett oder einer nano- oder mikrostrukturierten Oberfläche aus filamentärem oder tubulärem Material (z.B. Fiber/Hohlfiber).

Vorzugsweise besteht das Filtermaterial aus synthetischen oder natürlichen Polymeren, wie Polyvinylchlorid (PVC), Polyvinylidenfluorid (PVDF), Polypropylen (PP), Polyethersulfon (PES), Polyethylenglycol (PEG), Polyacrylamid (PAA), Polytetrafluorethylen (PTFE), Nylon, Polydimethylsiloxan (PDMS), Stärke, Zuckerverbindungen, Latex oder Silikon; Metall oder Metall-Legierungen, welche magnetisch, paramagnetisch, superpara-magnetisch oder unmagnetisch sein können; Glas, wie Borosilikatglas; Cellulose, wie Methylcellulose oder regenerierter Cellulose oder anderen Kohlenstoffverbindungen, wie Aktivkohle.

Die Filtration erfolgt vorzugsweise gravimetrisch, durch Anlegen eines Vakuums oder durch Überdruck.

Die Detektion von Bakterien-Bakteriophagen-Komplexen auf der Filteroberfläche im Retentat in Schritt D) sowie die Detektion von ungebundenen Testbakteriophagen im Filtrat in Schritt E) basiert jeweils auf der Detektion der Markierung, mit welcher die Bakteriophagen versehen sind. Beispielsweise kann die Markierung aus einem Reportermolekül mit fluoreszenten Eigenschaften bestehen. Nach entsprechender Anregung des Reportermoleküls, kann das emittierte Fluoreszenzsignal dann über einen Sensor detektiert werden. Die Fluoreszenzsignale können dabei entweder über einen optischen Emissions-Filter instantan integriert detektiert werden oder spektrophotometrisch als Wellenlängen-Spektrum vermessen werden. Ferner können beide Detektions-Strategien auch kombiniert werden. Der Vorteil der ersten Detektions-Strategie ist ein höheres Signal-Rausch-Verhältnis und eine schnellere Messung. Nachteil der ersten Detektions-Strategie ist eine eventuelle Einschränkung auf disjunkte Fluoreszenz-Marker, sofern mehrere unterschiedliche Testbakteriophagen verwendet werden. Der Vorteil der spektrophotometrischen Detektions-Strategie liegt darin, dass auch teilweise überlappende Markierungen bei mehreren unterschiedlichen Testbakteriophagen verwendet werden können, da diese im nachgeschalteten Prozessionsverfahren spektral entmischt und damit eindeutig zugeordnet werden können.

Die erhaltenen Signale aus den Schritten D) und E), wie beispielsweise die jeweils detektierten Fluoreszenzsignale und/oder Fluoreszenzspektren, werden in Schritt F) von einem Prozessor verarbeitet und die Ergebnisse der Detektion schließlich an einen Benutzer ausgegeben.

Die Ausgabe erfolgt vorzugsweise über ein mit dem Prozessor verbundenes optisches Display. Alternativ können die Detektionsergebnisse auch drahtlos per Funk von dem Prozessor an einen Tablet-Computer oder ein entferntes Computerterminal übertragen werden.

Bei Vorliegen mindestens einer nachzuweisenden Bakterienart in der Probe binden die markierten Testbakteriophagen der mindestens einen Spezies spezifisch an die mindestens eine nachzuweisende Bakterienart und bilden damit entsprechende Bakterien-Bakteriophagen-Komplexe. Durch die Filtration werden diese Komplexe sowie sonstige Bakterien von ungebundenen Bakteriophagen abgetrennt. Somit können die auf der Filteroberfläche immobilisierten Komplexe anhand der Markierung der gebundenen Testbakteriophagen detektiert werden. Die überschüssigen ungebundenen Testbakteriophagen können dien Filter passieren und anhand ihrer Markierung im Filtrat detektiert werden. Enthält die Probe keine nachzuweisende Bakterienart, so können sich keine Komplexe bilden und nur ungebundene Testbakteriophagen werden im Filtrat detektiert. Überschüssige ungebundene Testbakteriophagen können während des Verfahrens auch im Retentat nachgewiesen werden, insbesondere bei (teilweisem) Verstopfen des Filters. Zusätzlich zum Filtrat kann daher auch das Messsignal ungebundener Testbakteriophagen im Retentat in die Erfassung mit einbezogen werden.

Die zusätzliche Detektion von ungebundenen Testbakteriophagen im Schritt E) reduziert das Risiko, ein falsch positives Signal bei Verstopfen des Filters zu erhalten. Enthält die Probe keine nachzuweisende Bakterienart und wird der Filter beispielsweise durch eine nicht nachzuweisende Bakterienart oder andere Partikel in der Reaktionslösung verstopft, so können die ungebundenen Testbakteriophagen den Filter nicht passieren und verbleiben im Retentat. Daraus resultiert eine Detektion im Retentat, obwohl keine spezifischen Bakterien-Bakteriophagen-Komplexe gebildet werden können. Das Ergebnis ist ein falsch-positiver Nachweis der nachzuweisenden Bakterienart. Ist eine Detektion von ungebundenen Testbakteriophagen im Filtrat im Schritt E) also nicht möglich, so deutet dies auf ein Verstopfen des Filters hin. Folglich erhöht die zusätzliche Detektion von ungebundenen Testbakteriophagen im Schritt E) die Verlässlichkeit des erfindungsgemäßen Nachweisverfahrens.

Alternativ kann eine fehlende Detektion von ungebundenen Testbakteriophagen im Filtrat im Schritt E) darauf zurückzuführen sein, dass sämtliche Testbakteriophagen an die mindestens eine nachzuweisende Bakterienart gebunden haben. Wenngleich diese Möglichkeit aufgrund des hohen Phagentiters unwahrscheinlich ist, umfasst die Suspensionferner einen markierten Referenz-Bakteriophagen, welcher an keine nachzuweisende Bakterienart bindet und eine von den Testbakteriophagen disjunkte Markierung aufweist, um die Verlässlichkeit des erfindungsgemäßen Nachweisverfahrens weiter zu erhöhen. Durch die Markierung ist der Referenz-Bakteriophage ebenfalls detektierbar. Die Markierung des Referenz-Bakteriophagen ist dabei von den Markierungen der Testbakteriophagen disjunkt, so dass der Referenz-Bakteriophage eindeutig und unabhängig von den jeweiligen Testbakteriophagen detektiert werden kann. Da der Referenz-Bakteriophage an keine nachzuweisende Bakterienart bindet, d.h. keine Komplexe mit Bakterien bildet, kann er nach der Filtration, im Idealfall, ausschließlich im Filtrat detektiert werden. Ist dagegen kein Referenz-Bakteriophage im Filtrat detektierbar, so deutet dies auf ein vollständiges Verstopfen des Filters hin.

Auch bei teilweisem oder vollständigem Verstopfen des Filters während der Durchführung des Verfahrens liefert die Verwendung eines Referenz-Bakteriophagen ein sehr verlässliches Nachweisergebnis. Hierfür wird der Quotient aus Gesamtsignalintensität des Referenz-Bakteriophagen im Retentat und Gesamtsignalintensität des Referenz-Bakteriophagen im Filtrat ermittelt und mit dem Quotienten aus Gesamtsignalintensität des mindestens einen Testbakteriophagen im Retentat und Gesamtsignalintensität des mindestens einen Testbakteriophagen im Filtrat verglichen. Ist der ermittelte Quotient für den mindestens einen Testbakteriophagen dabei deutlich höher als der ermittelte Quotient für den Referenz-Bakteriophagen, so liefert dies einen eindeutigen Nachweis des Vorliegens mindestens einer nachzuweisenden Bakterienart in der verwendeten Probe. Finden sich dagegen Signale des mindestens einen Testbakteriophagen sowohl im Retentat als auch im Filtrat jedoch in vergleichbaren Quotienten wie für den Referenz-Bakteriophagen, so kann von einem unspezifischen Verstopfen des Filters, z.B. durch nicht nachzuweisende Bakterien, während der Durchführung des Verfahrens ausgegangen werden.

Alternativ zu einem markierten Referenz-Bakteriophagen werden markierte Mikro- oder Nanopartikel verwendet. Diese binden ebenfalls nicht an eine nachzuweisende Bakterienart und können den Filter aufgrund ihrer geringen Größe passieren.

Ein weiterer Vorteil des erfindungsgemäßen Verfahrens besteht darin, dass es nicht auf bestimmte nachzuweisende Bakterienarten beschränkt ist, sondern universell anwendbar ist. Einzige Voraussetzung ist das Vorhandensein eines komplementären Bakteriophagen zu einer nachzuweisenden Bakterienart. Gleich einem biologischen Prinzip, wonach in den zahlreichen Habitaten von Bakterienarten dort auch ihre komplementären Phagen vermutet werden, ist davon auszugehen, dass zu jeder Bakterienart mindestens ein Bakteriophage existiert, welche diese Bakterienart spezifisch infiziert.

Vorzugsweise werden die nachzuweisenden Bakterienarten jedoch ausgewählt aus der Gruppe umfassend *Staphylococcus aureus, Methicillin-resistente Staphylococcus aureus (MRSA), Staphylocossus epidermidis, Streptococcus pneumoniae, Streptococcus pyogenes, Haemophilus influenzae, Haemophilus parainfluenzae, Klebsiella pneumoniae, Escherichia coli, Escherichia hermannii, EHEC, Enterobacter cloacae, Enterococcus faecalis, Enterococcus faecum, Pseudomonas aeruginosa, Proteus mirabilis, Proteus vulgaris, Staphylococcus saprophyticus, Bacteroides fragilis, Entercoccus faecium, Enterobacter aerogenes, Serratia marcescens, B-Streptokokken (agalactiae), Chlamydia trachomatis, Chlamydia psittaci, Ureaplasma urealyticum, Mycoplasma hominis, Mycoplasma pneumoniae, Neisseria gonorrhoeae, Neisseria meningitidis, Citrobacter freundii, Moraxella catarrhalis, Stenotrophomonas maltophilia, Pasteurella multocida, Acinetobacter baumannii, Providencia rettgeri, Bordetella pertussis, Bacillus anthracis, Bacillus cereus, Brucella abortus, Brucella melitensis, Clostridium butolinum, Clostridium difficile, Clostridium tetani, Clostridium perfringens, Clamydia trachomatis, Corynebacterium diphtheriae, Francisella tularensis, Gardenella vaginalis, Listeria monocytogenes, Morganella morganii, Mycobacterium leprae, Mycobacterium tuberculosis, Nocardia asteriodes, Salmonella bongori, Salmonella enterica, Shigella spp., Vibrio cholerae, Borrelia burgdorferi, Yersinia pestis, Yersinia enterocolitica, Coxiella burnettii, Aeromonas spp., Plesiomonas spp., Xanthomonas maltophilia, Treponema pallidum, Eikenella corrodens, Spirillum minus, Rickettsia prowazeki, Rickettsia rickettsii, Rickettsia conorii, Cronobacter spp., Campylobacter spp. und Legionella pneumophilia.*

Besonders bevorzugt können die nachzuweisenden Bakterienarten ausgewählt sein aus der Gruppe bestehend aus *Staphylococcus aureus, Methicillin-resistente Staphylococcus aureus (MRSA), insbesondere haMRSA = hospitalaqcuired MRSA, caMRSA* = *Community-acquired MRSA, laMRSA = livestock associated MRSA, VRSA* = *Vancomycin-resistente Staphylococcus aureus, Staphylococcus epidermidis, Staphylococcus saprophyticus subsp. saprophyticus, Streptococcus pneumoniae, Streptococcus pyogenes, Streptococcus agalactiae, Streptococcus dysgalactiae, Enterococcus faecalis, Enterococcus faecium, Listeria monocytogenes, Clostridium difficile, Clostridium perfringens, Escherichia coli, Enterobacter cloacae, Enterobacter aerogenes, Haemophilus influenzae, Haemophilus parainfluenzae, Moraxella catarrhalis, Klebsiella pneumoniae, Klebsiella oxytoca, Serratia marcescens, Acinetobacter baumannii, Citrobacter freundii, Pseudomonas aeruginosa, Stenotrophomonas maltophilia, Proteus mirabilis, Proteus vulgaris, Morganella morganii, Providencia rettgeri, Providencia stuartii, Bacteroides fragilis, Chlamydia trachomatis, Ureaplasma urealyticum, Mycoplasma hominis, Neisseria gonorrhoeae, Treponema pallidum ssp. pallidum, Salmonella Typhi, Salmonella typhimurium, Pasteurella multocida, Shigella sonnei, Shigella flexneri, Campylobacter jejuni, Vibrio cholera, Francisella tularensis, Bordetella pertussis,* und *Legionella pneumophila.*

In einer Ausführungsform des erfindungsgemäßen Verfahrens umfasst die eine Suspension oder die mehreren Suspensionen vorzugsweise jeweils zwei oder mehrere Spezies von markierten Testbakteriophagen. Die Spezies binden jeweils spezifisch an unterschiedliche, nachzuweisende Bakterienarten. Vorzugsweise sind die Markierungen der Spezies disjunkt oder teilweise überlappend und somit für die jeweilige Spezies von Testbakteriophagen kennzeichnend. Dadurch wird ermöglicht, in einem Ansatz das Vorliegen zweier oder mehrerer Bakterienarten in einer Probe gleichzeitig nachzuweisen. Vorzugweise enthält die eine Suspension oder die mehreren Suspensionen jeweils bis zu 6 verschiedene Spezies, weiter vorzugsweise bis zu 5 verschiedene Spezies, noch weiter vorzugsweise bis zu 4 verschiedene Spezies und am meisten bevorzugt bis zu 3 verschiedene Spezies von markierten Testbakteriophagen.

In einer weiteren Ausführungsform wird das erfindungsgemäße Verfahren parallel mit zwei oder mehreren Suspensionen durchgeführt, welche jeweils eine andere Spezies von markierten Testbakteriophagen umfassen, wobei die Markierungen der Spezies vorzugsweise disjunkt oder teilweise überlappend sind. Die zu testende Probe wird dabei auf die zwei oder mehreren Suspensionen verteilt. Vorzugsweise erfolgt die Verteilung der zu testenden Probe über ein Mikrofluidik-System, so dass die Probe nicht einzeln zu den Suspensionen gegeben werden muss, sondern die Zugabe gleichzeitig durch Mikrofluidik-Kanäle des Mikrofluidik-Systems erfolgt. Vorzugsweise werden in dieser Ausführungsform 6 parallele Suspensionen, weiter vorzugsweise 5 parallele Suspensionen, noch weiter vorzugsweise 4 parallele Suspensionen und am meisten bevorzugt bis zu 3 parallele Suspensionen verwendet. Dadurch wird ebenfalls ermöglicht, das Vorliegen zweier oder mehrerer Bakterienarten in einer Probe gleichzeitig nachzuweisen.

In einer anderen Ausführungsform umfasst das Verfahren ferner einen Schritt, welcher auf Schritt A) folgt, in welchem die markierten Bakteriophagen in der Suspension vor Zugabe der Probe detektiert werden. Dieser Schritt dient der Erfassung der Gesamtheit der Signale, welche von den Markierungen der ungebundenen Bakteriophagen stammen, als Ausgangsreferenz. Handelt es sich bei der Markierung beispielsweise um ein Reportermolekül mit fluoreszenten Eigenschaften, so wird nach entsprechender Anregung die Gesamtfluoreszenz der ungebundenen Bakteriophagen als Ausgangsreferenz vor Zugabe der Probe detektiert bzw. dessen Fluoreszenzspektrum mittels eines Spektral-Photometers aufgezeichnet.

Vorzugsweise umfasst das Verfahren weiterhin die Detektion der in Schritt B) bzw. der im optionalen Schritt G) entstandenen Reaktionslösung vor der Filtration.

Die Markierung der Bakteriophagen umfasst vorzugsweise mindestens ein Reportermolekül mit fluoreszenten oder chemilumineszenten Eigenschaften oder mindestens ein Reportermolekül, welches durch Interaktion mit einem sekundären Molekül Licht emittiert. Weiter bevorzugt ist mindestens ein Reportermolekül mit fluoreszenten oder chemilumineszenten Eigenschaften. Mindestens ein Reportermolekül mit fluoreszenten Eigenschaften ist besonders bevorzugt.

Zu den Reportermolekülen mit fluoreszenten Eigenschaften zählen
- organische Fluorophore, wie Xanthene, Rhodamine, Cumarine, AcridinDerivate, Fluorescein- und Alexa Fluor-Derivate, SYBR Green und SYBR Gold;
- anorganische Fluorophore und deren Komposite, wie Quantenpunkte (quantum dots, QD);
- fluoreszierende magnetische Partikel; und
- fluoreszierende Proteine, wie das grün-fluoreszierende Protein (GFP), das gelb-fluoreszierende Protein (YFP) oder Derivate davon.

Zu den Reportermolekülen, welche durch Interaktion mit einem sekundären Molekülen Licht emittiert, zählen
- Enzyme, wie Alkalische Phosphatase oder Meerrettichperoxidase, welche durch Umsatz entsprechender Markersubstrate Licht emittieren; und
- Biotin, das durch Bindung von Streptavidin als Interaktionspartner, welches wiederum eine fluoreszente oder chemilumineszente Markierung trägt, Licht emittiert.

Für die Markierung der Bakteriophagen sind organische Fluorophore am meisten bevorzugt.

Die Markierung der Bakteriophagen kann durch Kopplung mindestens eines Reportermoleküls an die DNA des Phagen, an Proteine im Capsid-Bereich (Kopfhüllenproteine) oder an Proteine im Schwanz-Bereich des Phagens (*tail fiber* Proteine) erfolgen. Dabei ist die Kopplung mindestens eines Reportermoleküls an Proteine im Capsid-Bereich der Bakteriophagen bevorzugt.

Vorzugsweise erfolgt die Kopplung durch Ausbildung einer kovalenten Bindung zwischen dem mindestens einen Reportermolekül und dem Bakteriophagen. Hierzu werden verwendet
- Aktivester, wie N-Hydroxysuccinimidester (NHS-ester) oder Sulfotetrafluorophenylester (STFP-ester), des mindestens einen Reportermoleküls zur Kopplung an Aminogruppen;
- Isothiocyanate des mindestens einen Reportermoleküls zur Kopplung an Aminogruppen;
- Maleimide des mindestens einen Reportermoleküls zur Kopplung an Thiolgruppen;
- Azide des mindestens einen Reportermoleküls zur Kopplung über eine zweistufige Azid-Alkin-Cycloadditionsreaktion an Alkine; und
- Photoreaktive Gruppen in dem mindestens einem Reportermolekül zur unspezifischen Kopplung.

Exemplarisch seien 5(6)-Carboxyfluorescein-NHS-ester als Beispiel eines Aktivesters, Fluoresceinisothiocyanat (FITC) als Beispiel eines Isothiocyanats, Alexa Fluor 488-maleimid als Beispiel eines Maleimids, 5-Azidotetramethylrhodamin (5-TAMRA-azid) als Beispiel eines Azids und Photobiotin als Beispiel einer photoreaktiven Gruppe genannt.

In einer bevorzugten Ausführungsform des erfindungsgemäßen Verfahrens besteht die Markierung der Bakteriophagen jeweils aus mindestens einem fluoreszenten Reportermolekül. Die Anregung erfolgt jeweils durch eine Beleuchtungseinheit, welche vorzugsweise einen oder mehrere Laser oder eine oder mehrere Licht-emittierende Dioden (LED) umfasst. Ferner erfolgt die Anregung im Wellenlängenbereich von 200-1000 nm, weiter vorzugsweise im Wellenlängenbereich von 350-700 nm. Die Detektion von Bakterien-Bakteriophagen-Komplexen auf der Filteroberfläche im Retentat sowie die Detektion von ungebundenen Bakteriophagen im Filtrat erfolgt jeweils durch Messung der über einen optischen Emissionsfilter integrierten Fluoreszenz mittels eines optischen Sensors und/oder durch Messung des Fluoreszenzintensitätsspektrums. Die Messung der Fluoreszenz erfolgt vorzugsweise fluoreszenzmikroskopisch und/oder mit Hilfe eines optischen Sensors und/oder mittels eines Spektral-Photometers. Der optische Sensor wird weiter vorzugsweise ausgewählt wird aus der Gruppe bestehend aus einem Photomultiplier (PMT), einem CCD-Sensor (*charge coupled device*) und einem CMOS-Sensor (*complementary metal-oxide-semiconductor*). Das Spektral-Photometer erlaubt die Abtastung der Fluoreszenz-Intensität in vorgegebenen Wellenlängen-Intervallen, vorzugsweise in 20 nm Schritten, weiter bevorzugt in 10 nm Schritten, insbesondere bevorzugt in 2-5 nm Schritten.

In einer weiteren bevorzugten Ausführungsform erfolgt die Detektion jeweils durch zeitaufgelöste Messung der Lichtemission, d.h. das jeweils von Reportermolekülen emittierte Licht wird über einen bestimmten Zeitraum kontinuierlich detektiert. Handelt es sich bei der Markierung der Bakteriophagen jeweils um mindestens ein fluoreszentes Reportermolekül, so erfolgt die Anregung und Detektion wie in der vorhergehenden Ausführungsform beschrieben, mit der Maßgabe, dass die Fluoreszenz zeitaufgelöst gemessen wird.

Vorzugsweise umfasst das erfindungsgemäße Verfahren zum Nachweis von Bakterien ferner den Schritt einer Vortrennung von Bakterien-Bakteriophagen-Komplexen und ungebundenen Bakteriophagen, wobei sich die Vortrennung an Schritt B) bzw. an den optionalen Schritt G) anschließt. Dabei erfolgt die Vortrennung des Reaktionsgemisches vorzugsweise nach dem Prinzip der Gelfiltration (Gelpermeationschromatographie bzw. Größenausschlussvolumen-Chromatographie) durch eine Gelmatrix. Dies bedeutet, dass Bakterien-Bakteriophagen-Komplexe und ungebundene Bakteriophagen aufgrund ihres Größenunterschiedes separiert werden, wobei die größeren Komplexe die Gelmatrix vor den kleineren ungebundenen Bakteriophagen verlassen. Die Gelmatrix besteht vorzugsweise aus synthetischen oder natürlichen Polymeren, wie Polyvinylchlorid (PVC), Polyvinylidenfluorid (PVDF), Polypropylen (PP), Polyethersulfon (PES), Polyethylenglycol (PEG), Polyacrylamid (PAA), Polytetrafluorethylen (PTFE), Nylon, Polydimethylsiloxan (PDMS), Stärke, Zuckerverbindungen, Latex oder Silikon; Metall oder Metall-Legierungen, welche magnetisch, paramagnetisch, superparamagnetisch oder unmagnetisch sein können; Glas, wie Borosilikatglas; Cellulose, wie Methylcellulose oder regenerierter Cellulose oder anderen Kohlenstoffverbindungen.

Die Gelmatrix weist vorzugsweise eine Porengröße von 0,01-5 µm auf. Die Wahl des Materials sowie der Porengröße der Gelmatrix hängt dabei von der Größe und Morphologie der mindestens einen nachzuweisenden Bakterienart ab, so dass jeweils eine optimale Vortrennung der Bakterien-Bakteriophagen-Komplexe von ungebundenen Bakteriophagen erzielt wird.

In einer weiteren Ausführungsform umfasst das erfindungsgemäße Verfahren ferner den Schritt einer Konzentrierung der Bakterien-Bakteriophagen-Komplexe und der ungebundenen Bakteriophagen vor der Filtration in Schritt C).

Die Konzentrierung kann mechanisch oder elektromagnetisch erfolgen.

Weiter vorzugsweise sind die Testbakteriophagen der mindestens einen Spezies sowie der Referenzphage mit magnetischen Beads beschichtet. In diesem Fall kann die Konzentrierung elektromagnetisch durch Anlegen eines externen Magnetfeldes erfolgen, beispielsweise durch Bereitstellen eines Elektromagneten.

Die optionalen Schritte der Vortrennung und der Konzentrierung können jeweils einzeln oder auch in Kombination in dem erfindungsgemäßen Verfahren durchgeführt werden.

In einer weiteren Ausführungsform kann das erfindungsgemäße Verfahren einen Spülschritt nach der Filtration des Reaktionsgemisches im Schritt C) umfassen. In diesem Spülschritt wird vorzugsweise der Filter mit Neutrallösung gespült. Die Neutrallösung besteht vorzugsweise aus Wasser, einer Pufferlösung oder sonstigen wässrigen Lösung oder aus einer zuvor sterilfiltrierten Probe.

In einem weiteren Aspekt beschreibt die vorliegende Erfindung ein Reaktionsgefäß zur Durchführung des erfindungsgemäßen Nachweises von Bakterien.

Vorzugsweise weist das erfindungsgemäße Reaktionsgefäß eine Länge von 5-20 mm, eine Breite von 5-20 mm und eine Höhe von 50-100 mm auf.

Das erfindungsgemäße Reaktionsgefäß umfasst mindestens zwei Kompartimente, welche durch einen Filter voneinander getrennt sind. Der Filter weist vorzugsweise eine Porengröße von 0,1 µm bis 0,5 µm, weiter vorzugsweise von 0,2-0,5 µm auf. Besonders bevorzugt wird eine Porengröße von 0,3-0,5 µm und am meisten bevorzugt ist eine Porengröße von 0,2-0,4 µm. Wie zuvor für das Verfahren beschrieben, wird die Porengröße des verwendeten Filters entsprechend der Größe und Morphologie der jeweiligen nachzuweisenden Bakterienart gewählt, um eine effiziente Trennung von Bakterien-Bakteriophagen-Komplexen und sonstigen eventuell vorliegenden Bakterien von ungebundenen Bakteriophagen zu erreichen.

Der Filter besteht vorzugsweise aus einer Membran, einer Folie oder einer Matrix, wie einem Kugelbett oder einer nano- oder mikrostrukturierten Oberfläche aus filamentärem oder tubulärem Material (z.B. Fiber/Hohlfiber).

Vorzugsweise besteht das Filtermaterial aus synthetischen oder natürlichen Polymeren, wie Polyvinylchlorid (PVC), Polyvinylidenfluorid (PVDF), Polypropylen (PP), Polyethersulfon (PES), Polyethylenglycol (PEG), Polyacrylamid (PAA), Polytetrafluorethylen (PTFE), Nylon, Polydimethylsiloxan (PDMS), Stärke, Zuckerverbindungen, Latex oder Silikon; Metall oder Metall-Legierungen, welche magnetisch, paramagnetisch, superparamagnetisch oder unmagnetisch sein können; Glas, wie Borosilikatglas; Cellulose, wie Methylcellulose oder regenerierter Cellulose oder anderen Kohlenstoffverbindungen, wie Aktivkohle.

Ein Kompartiment des Reaktionsgefäßes ist ein Phagenreservoir, welches eine Suspension enthält, die mindestens eine Spezies von markierten Testbakteriophagen umfasst, welche spezifisch an eine nachzuweisende Bakterienart bindet. Vorzugsweise ist die Markierung für die jeweilige Spezies von Testbakteriophagen kennzeichnend.

In einer weiteren Ausführungsform umfasst die Suspension im Phagenreservoir zwei oder mehrere Spezies von markierten Testbakteriophagen, welche jeweils spezifisch an unterschiedliche nachzuweisende Bakterienarten binden, wobei die Markierungen der Spezies disjunkt oder teilweise überlappend sind, so dass die Markierung für die jeweilige Spezies von Testbakteriophagen kennzeichnend ist.

Die Konzentration der Bakteriophagen in der Suspension, das Volumen der Suspension sowie die weiteren Spezifikationen der Suspension entsprechen den Angaben wie für das erfindungsgemäße Verfahren beschrieben.

Ebenso gelten die Angaben des Verfahrens bezüglich der markierten Testbakteriophagen und deren Markierung entsprechend für das erfindungsgemäße Reaktionsgefäß.

Die Suspension im Phagenreservoir umfasst ferner einen markierten Referenz-Bakteriophagen, welcher an keine nachzuweisende Bakterienart bindet und eine von den Testbakteriophagen disjunkte Markierung aufweist. Die vorteilhaften Wirkungen dieser Ausführungsform sind im Detail für das erfindungsgemäße Verfahren beschrieben.

In einer Ausführungsform des erfindungsgemäßen Reaktionsgefäßes ist das Phagenreservoir durch eindrückbare Membranen geschlossen, so dass die Suspension nicht in direktem Kontakt mit dem Filter steht. Erst durch Eindrücken der eindrückbaren Membranen bei der Zugabe einer Probe, die auf das Vorliegen mindestens einer nachzuweisenden Bakterienart getestet werden soll, fließt die entstehende Reaktionslösung auf den Filter.

Ein Kompartiment des erfindungsgemäßen Reaktionsgefäßes ist ein Auffangreservoir, welches für die Aufnahme des Filtrats der Reaktionslösung nach Zugabe einer Probe zu der Suspension und anschließender Filtration vorgesehen ist.

Weiterhin umfasst das Reaktionsgefäß ein Mittel zu dessen Identifikation.

Vorzugsweise handelt es sich dabei um einen RFID-Chip (*radio-frequency identification*) oder um einen Barcode. Weiter vorzugsweise enthalten der RFID-Chip (*radio-frequency identification*) oder der Barcode Daten hinsichtlich der Seriennummer, des Produktionsdatums, des Ablaufdatums, des Phagentiters in der Suspension und/oder der nachzuweisenden Bakterienart(en) bzw. der nachzuweisenden Stämme. Das Mittel zur Identifikation ermöglicht eine einfache und eindeutige Identifizierung des Reaktionsgefäßes, wodurch die Gefahr von Verwechslungen von Reaktionsgefäßen minimiert und somit die Verlässlichkeit eines damit durchgeführten Nachweisverfahrens erhöht wird.

Weiterhin umfasst das erfindungsgemäße Reaktionsgefäß mindestens zwei Messfenster, wobei ein Messfenster im Bereich der Filteroberfläche, welche dem Phagenreservoir zugewandt ist, angeordnet ist, und ein Messfenster im Bereich des Auffangreservoirs angeordnet ist. Dadurch wird die Detektion von Bakterien-Bakteriophagen-Komplexen auf der Filteroberfläche im Retentat und die Detektion von ungebundenen Bakteriophagen im Filtrat bei der Durchführung des erfindungsgemäßen Nachweisverfahrens unter Verwendung des erfindungsgemäßen Reaktionsgefäßes ermöglicht. Das erfindungsgemäße Reaktionsgefäß kann im Bereich der Messfenster zusätzlich Anregungsfenster umfassen, welche eine Anregung von Reportermolekülen mit fluoreszenten Eigenschaften ermöglichen. Alternativ kann die Anregung jeweils auch durch die Messfenster selbst erfolgen.

In einer bevorzugten Ausführungsform umfasst das Reaktionsgefäß ein weiteres Kompartiment zwischen Phagenresevoir und Filter, welches mindestens einen Vorfilter enthält. Der mindestens eine Vorfilter ermöglicht eine Vortrennung von Bakterien-Bakteriophagen-Komplexen und ungebundenen Bakteriophagen aus dem Reaktionsgemisch vorder Filtration.

Weiter vorzugsweise handelt es sich bei dem Vorfilter um eine Gelmatrix, welche das Reaktionsgemisch nach dem Prinzip der Gelfiltration vortrennt. Die Gelmatrix besteht vorzugsweise aus synthetischen oder natürlichen Polymeren, wie Polyvinylchlorid (PVC), Polyvinylidenfluorid (PVDF), Polypropylen (PP), Polyethersulfon (PES), Polyethylenglycol (PEG), Polyacrylamid (PAA), Polytetrafluorethylen (PTFE), Nylon, Polydimethylsiloxan (PDMS), Stärke, Zuckerverbindungen, Latex oder Silikon; Metall oder Metall-Legierungen, welche magnetisch, paramagnetisch, superparamagnetisch oder unmagnetisch sein können; Glas, wie Borosilikatglas; Cellulose, wie Methylcellulose oder regenerierter Cellulose oder anderen Kohlenstoffverbindungen. Ferner weist die Gelmatrix vorzugsweise eine Porengröße von 0,01-5 µm auf. Die Wahl des Materials sowie der Porengröße der Gelmatrix hängt dabei von der Größe und Morphologie der mindestens einen nachzuweisenden Bakterienart ab, so dass jeweils eine optimale Vortrennung der Bakterien-Bakteriophagen-Komplexe von ungebundenen Bakteriophagen erzielt wird.

In dieser Ausführungsform umfasst das Reaktionsgefäß weiterhin ein oder mehrere Messfenster, die unmittelbar unterhalb des Kompartiments, welches mindestens einen Vorfilter enthält, angeordnet sind.

Vorzugsweise verengt sich das Reaktionsgefäß vom Phagenreservoir zum Filter hin, so dass eine Konzentrierung der Bakterien-Bakteriophagen-Komplexe und der ungebundenen Bakteriophagen vor der Filtration ermöglicht wird.

Darüber hinaus wird das Reaktionsgefäß vorzugsweise im Bereich des Phagenreservoirs durch ein Septum verschlossen.

Das Reaktionsgefäß umfasst vorzugsweise ein weiteres Messfenster, welches im Bereich des Phagenreservoirs angeordnet ist. Dies ermöglicht die Detektion der Gesamtheit der Signale, welche von den Markierungen der ungebundenen Bakteriophagen stammen, als Ausgangsreferenz. Handelt es sich bei der Markierung beispielsweise um ein Reportermolekül mit fluoreszenten Eigenschaften, so wird nach entsprechender Anregung die Gesamtfluoreszenz der ungebundenen Bakteriophagen als Ausgangsreferenz vor Zugabe der Probe detektiert bzw. dessen Fluoreszenzspektrum mittels eines Spektral-Photometers aufgezeichnet.

Weiterhin umfasst das Reaktionsgefäß vorzugsweise ein oder mehrere Kompartimente, die sich an das Auffangreservoir anschließen und als Folgeaufkonzentrierungsreservoir(e) bezeichnet werden. Das letzte dieser Kompartimente wird als Abfallreservoir bezeichnet. Weiter vorzugsweise werden die ein oder mehreren Folgeaufkonzentrierungsreservoir(e) durch Folgefilter mit einer Porengröße von 0,1 µm bis 0,5 µm, vorzugsweise von 0,2-0,5 µm, weiter vorzugsweise von 0,3-0,5 µm und am meisten bevorzugt von 0,2-0,4 µm, von dem Auffangreservoir bzw. voneinander getrennt. Die Folgeaufkonzentrierungsreservoire können ebenfalls mit eigenen Messfenstern und Anregungsfenstern versehen sein. Ferner kann die Porengröße der Folgefilter vom Auffangreservoir zum Abfallreservoir hin abnehmen, was eine serielle Trennung von verschiedenen Bakterien und Bakterien-Bakteriophagen-Komplexen entsprechend der Größe und Morphologie der jeweiligen nachzuweisenden Bakterienart ermöglicht. Das Abfallreservoir wird durch einen Endfilter mit einer Porengröße von ≤ 0,05 µm vom Auffangreservoir oder, sofern vorhanden, von dem einen oder den mehreren Folgeaufkonzentrierungsreservoir(en) getrennt. Durch den Endfilter werden sämtliche Bakteriophagen entfernt, so dass das verbleibende Filtrat kostengünstig und gefahrlos entsorgt werden kann. Alternativ enthält das Abfallreservoir ein Absorptionsmittel, welches das Filtrat aus dem Auffangreservoir oder, sofern vorhanden, aus dem einen oder den mehreren Folgeaufkonzentrierungsreservoir(en) absorbiert.

In einer alternativen Ausführungsform umfasst das Reaktionsgefäß ein weiteres Kompartiment zwischen Filter und Phagenreservoir, welches vorzugsweise von anderen Kompartimenten durch ein Septum getrennt ist. Dieses Kompartiment enthält eine Indikatorlösung mit markierten Mikro- oder Nanopartikeln, welche nicht an eine nachzuweisende Bakterienart binden und den Filter aufgrund ihrer geringen Größe passieren können. Daher kann die Indikatorlösung mit den markierten Mikro- oder Nanopartikeln als Alternative zu einem Referenz-Bakteriophagen verwendet werden und mittels Durchstechen der Membran zugeben werden.

Die verschiedenen Ausführungsformen des erfindungsgemäßen Reaktionsgefäßes schließen sich gegenseitig nicht aus und können daher einzeln oder in Kombination verwirklicht werden.

In einem weiteren Aspekt bezieht sich die vorliegende Erfindung auf eine Kartusche, welche zwei oder mehrere erfindungsgemäße Reaktionsgefäße umfasst, die parallel und/oder seriell zueinander angeordnet sind, wobei jedes parallele Reaktionsgefäß unterschiedliche Spezies von markierten Testbakteriophagen in der Suspension in den Phagenreservoirs enthält, welche jeweils spezifisch an unterschiedliche nachzuweisende Bakterienarten binden. Dadurch wird ermöglicht, das Vorliegen zweier oder mehrerer Bakterienarten in einer Probe gleichzeitig nachzuweisen. Die Markierungen der unterschiedlichen Spezies von markierten Testbakteriophagen sind vorzugsweise disjunkt oder teilweise überlappend. Die serielle Anordnung von Reaktionsgefäßen ermöglicht eine Trennung von verschiedenen Bakterien und Bakterien-Bakteriophagen-Komplexen entsprechend der Größe und Morphologie der jeweiligen nachzuweisenden Bakterienarten. In diesem Fall ist die Porengröße des Filters des nachgeschalteten Reaktionsgefäßes bzw. der nachgeschalteten Reaktionsgefäße jeweils kleiner als die Porengröße des Filters im vorhergehenden Reaktionsgefäß zu wählen.

Vorzugsweise umfasst die Kartusche bis zu 6 parallel angeordnete Reaktionsgefäße, weiter vorzugsweise bis zu 5 parallel angeordnete Reaktionsgefäße, noch weiter vorzugsweise bis zu 4 parallel angeordnete Reaktionsgefäße und am meisten bevorzugt bis zu 3 parallel angeordneten Reaktionsgefäße.

Darüber hinaus umfasst die Kartusche vorzugsweise ein Mikrofluidik-System, in welches die zu testende Probe injiziert wird und welches die Probe über Mikrofluidik-Kanäle jeweils zu den Phagenreservoirs der parallel angeordneten Reaktionsgefäße leitet, so dass die Probe nicht einzeln zu den Suspensionen gegeben werden muss.

Die vorliegende Erfindung betrifft weiterhin die Verwendung eines Messgeräts zur Durchführung des erfindungsgemäßen Verfahrens zum Nachweis von Bakterien.

Das Messgerät umfasst einen Schacht, in den ein erfindungsgemäßes Reaktionsgefäß oder eine erfindungsgemäße Kartusche eingeschoben wird.

Ferner umfasst das Messgerät mindestens zwei Detektions-Optiken, welche jeweils im Bereich der Messfenster des eingeschobenen Reaktionsgefäßes oder der Kartusche angeordnet sind und einen Prozessor.

Umfasst das Messgerät einen Schacht für ein Reaktionsgefäß, so kann es je nach Ausführungsform des Reaktionsgefäßes, d.h. entsprechend der Anzahl der Messfenster, drei, vier oder mehr Detektions-Optiken beinhalten.

Umfasst das Messgerät dagegen einen Schacht für eine Kartusche, so hängt die Anzahl der Detektions-Optiken von der Anzahl der in Kartusche parallel und/oder sequentiell angeordneten Reaktionsgefäße ab, wobei das Messgerät pro parallel angeordnetem Reaktionsgefäß zwei bis vier oder mehr Detektions-Optiken beinhalten kann.

Die Detektions-Optiken umfassen jeweils ein Linsensystem zur Fokussierung von emittiertem Licht der Markierung der Bakteriophagen auf mindestens einen Sensor, wobei der mindestens eine Sensor das emittierte Licht detektiert. Der mindestens eine Sensor der Detektions-Optiken umfasst vorzugsweise einen oder mehrere optische Sensoren und/oder ein oder mehrere Spektral-Photometer, welche mit oder ohne Emissions-Filter bestückt sind, wodurch die Auflösung des Spektrums von Fluoreszenz-Signalen ermöglicht wird. Im Falle einer Kombination von optischem Sensor und einem oder mehreren Spektral-Photometern können die Detektions-Optiken sequentiell angesteuert werden und so zwischen schneller Detektion mit hohem Signal-Rausch-Verhältnis (linsenfokussierte Detektion der Gesamt-Fluoreszenzintensität im optischen Sensor) oder langsamer Detektion mit Aufschlüsselung des Spektrums (Spektral-Photometer) gewechselt werden. Ein Spektral-Photometer wird hierbei nicht als optischer Sensor im strengeren Sinne verstanden.

Vorzugsweise werden die optischen Sensoren ausgewählt aus der Gruppe bestehend aus einem Photomultiplier (PMT), einem CCD-Sensor (*charge coupled device*) und einem CMOS-Sensor (*complementary metal-oxide-semiconductor*).

Darüber hinaus umfassen die Detektions-Optiken vorzugsweise jeweils eine Beleuchtungseinheit, welche Licht im Wellenlängenbereich von 200-1000 nm emittiert und ein weiteres Linsensystem zur Fokussierung des von der Beleuchtungseinheit emittierten Lichts auf einen Messbereich. Die Beleuchtungseinheit ist dabei ebenfalls mit dem Prozessor verbunden und wird von diesem gesteuert.

Die Beleuchtungseinheit emittiert vorzugsweise Licht im Wellenlängenbereich von 350-700 nm. Weiter vorzugsweise umfasst die Beleuchtungseinheit einen Laser oder eine oder mehrere Licht-emittierende Dioden (LED).

Der Prozessor ist jeweils mit dem mindestens einen Sensor der Detektionsoptiken verbunden und verarbeitet die erhaltenen Detektionssignale.

Darüber hinaus umfasst das Messgerät eine mit dem Prozessor verbundene Ausgabeeinheit, welche die Detektionsergebnissen an einen Benutzer ausgibt.

Die Ausgabeeinheit umfasst vorzugsweise ein optisches Display.

Weiter vorzugsweise umfasst die Ausgabeeinheit eine Funkschnittstelle, wodurch die Detektionsergebnisse drahtlos per Funk von dem Prozessor an einen Tablet-Computer oder ein entferntes Computerterminal übertragen werden können.

Vorzugsweise umfasst das Messgerät ferner ein integriertes Lesegerät, welches das Mittel zur Identifikation des Reaktionsgefäßes erfasst und die erfassten Daten an den Prozessor weiterleitet, wobei der Prozessor die erfassten Daten verarbeitet und die Ergebnisse der Identifikation über die Ausgabeeinheit an einen Benutzer ausgegeben werden.

In einer bevorzugten Ausführungsform umfasst das Messgerät ferner einen selektiv zu und abschaltbaren Elektromagneten, wie beispielsweise eine Ringspule. Dieser ist weiter vorzugsweise im Bereich der Filteroberfläche des in den Schacht eingeschobenen Reaktionsgefäßes angeordnet, welche dem Phagenreservoir zugewandt ist.

Im Folgenden wird die vorliegende Erfindung unter Bezugnahme auf die Figuren im Detail dargestellt und verschiedene Ausführungsformen weiter erläutert.

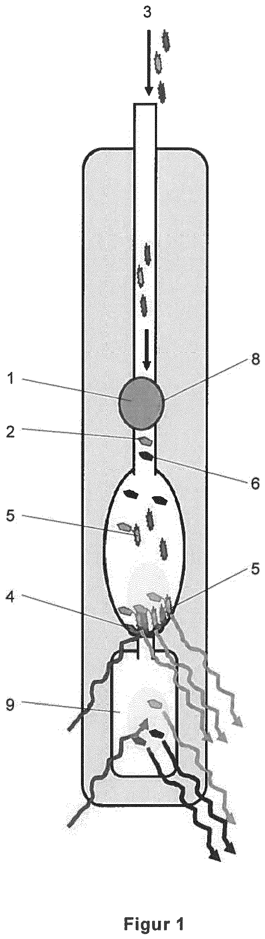

Figur 1 zeigt eine Ausführungsform des erfindungsgemäßen Verfahrens. Dabei wird eine Probe (3), welche auf das Vorliegen einer nachzuweisenden Bakterienart getestet werden soll, in eine Suspension (1), welche in einem eindrückbaren Phagenreservoir (8) bereitgestellt ist, injiziert. Die Probe (3) kann dabei weitere, nicht nachzuweisende Bakterienarten enthalten. Die Suspension (1) enthält in dieser Ausführungsform eine Spezies von markierten Testbakteriophagen (2), welche spezifisch an die nachzuweisende Bakterienart bindet. Bei der Markierung handelt es sich um einen Reportermolekül mit fluoreszenten Eigenschaften. Nach Mischen der Probe (3) mit der Suspension (1) und Inkubation des Reaktionsgemisches fließt dieses auf den Filter (4) mit einer bevorzugten Porengröße zwischen 0,1 µm und 0,5 µm und wird gravimetrisch und/oder unter Einwirkung eines äußeren Stempeldrucks oder Vakuums filtriert (,dead end filtration'). Dadurch werden die entstandenen Bakterien-Bakteriophage-Komplexe (5) sowie weitere, nicht nachzuweisende Bakterienarten auf der Filteroberfläche im Retentat immobilisiert, während die verbliebenen ungebundenen Testbakteriophagen (2) die Poren des Filters (4) passieren und als Filtrat in einem Auffangreservoir (9) gesammelt werden. Die Detektion der Bakterien-Bakteriophagen-Komplexe (5) auf der Filteroberfläche sowie der ungebundenen Testbakteriophagen (2) im Filtrat erfolgt durch spezifische Anregung der verwendeten fluoreszenten Reportermoleküle und durch Messung der resultierenden Fluoreszenz. In der Regel erfolgt die Anregung in dem Wellenlängenbereich, in welchem das Absorptionsmaximum des jeweiligen fluoreszenten Reportermoleküls liegt. In dieser Ausführungsform enthält die Suspension (1) ferner einen markierten Referenz-Bakteriophagen (6), welcher nicht an die nachzuweisende Bakterienart bindet, dadurch die Poren des Filters (4) passiert und ebenfalls als Filtrat in dem Auffangreservoir (9) aufgefangen wird. Die Markierung des Referenz-Bakteriophagen (6) besteht dabei ebenfalls aus einem Reportermolekül mit fluoreszenten Eigenschaften, wobei die Reportermoleküle der Test- (2) und des Referenzbakteriophagen (6) vorzugsweise disjunkt sind. Dies bedeutet, dass die jeweiligen fluoreszenten Reportermoleküle derart ausgewählt werden, dass sie Licht bei gleichen oder unterschiedlichen Anregungswellenlängen absorbieren können, jedoch Licht bei unterschiedlichen Wellenlängen emittieren. Dadurch können die emittierten Fluoreszenzsignale der jeweiligen Reportermoleküle und damit der jeweiligen Bakteriophagen eindeutig unterschieden werden. Die Detektion des Referenz-Bakteriophagen (6) erfolgt dementsprechend ebenfalls durch spezifische Anregung seiner fluoreszenten Reportermoleküle und durch Messung der resultierenden Fluoreszenz. Die erhaltenen Fluoreszenzsignale aus dem Retentat und dem Filtrat werden anschließend von einem Prozessor verarbeitet und die Ergebnisse der Detektion an einen Benutzer ausgegeben.

Bei spektrophotometrischer Detektion der Fluoreszenz, d.h. bei Verwendung eines Spektral-Photometers als Sensor, kann eine weitere Detektion über einen einstellbar spektralbandigen Wellenlängenbereich durchgeführt werden, um das Emissionsspektrum zu erfassen. In diesem Fall können anstatt disjunkter Fluoreszenz-Reportermoleküle auch überlappende Fluoreszenz-Reportermoleküle verwendet werden, da diese im Multiplexing-Verfahren detektiert und spektral nachgeschaltet prozessorgesteuert entmischt werden können.

Die Ausgabe kann über ein, mit dem Prozessor verbundenes optisches Display oder drahtlos per Funk an einen Tablet-Computer oder ein entferntes Computerterminal erfolgen. Anhand der Bildung der Quotienten aus Gesamtintensität der gemessenen Fluoreszenz im Retentat und Gesamtintensität der gemessenen Fluoreszenz im Filtrat für den Test- (2) und für den Referenz-Bakteriophagen (6) und Vergleich dieser lässt sich ein verlässliches Ergebnis des Nachweisverfahrens erzielen. Ist der ermittelte Quotient für den Testbakteriophagen (2) dabei deutlich höher als der ermittelte Quotient für den Referenz-Bakteriophagen (6), so liefert dies einen eindeutigen Nachweis des Vorliegens der nachzuweisenden Bakterienart in der verwendeten Probe. Finden sich dagegen Fluoreszenzsignale des Testbakteriophagen (2) sowohl im Retentat als auch im Filtrat, jedoch im vergleichbaren Verhältnis wie für den Referenz-Bakteriophagen (6), so kann von einem unspezifischen Verstopfen des Filters (4), z.B. durch nicht nachzuweisende Bakterien, während der Durchführung des Verfahrens ausgegangen werden.

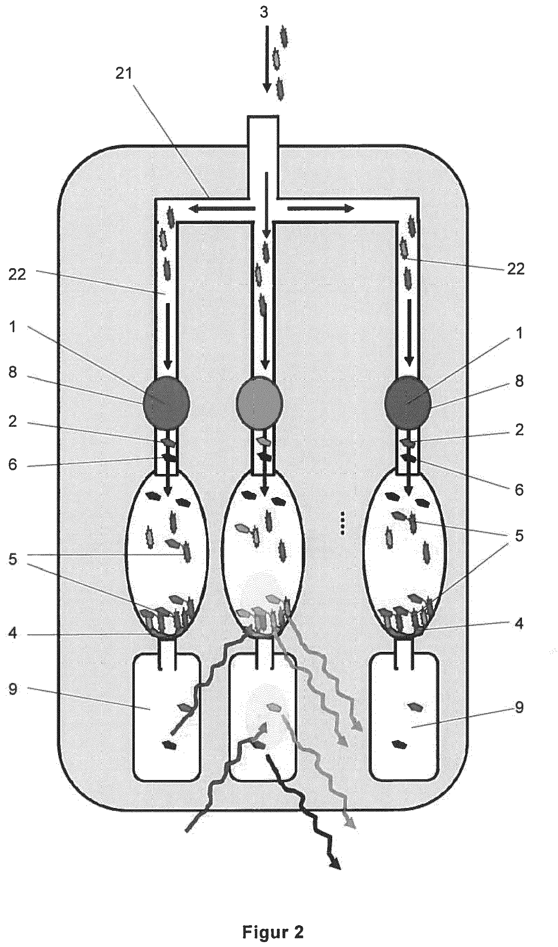

Eine weitere Ausführungsform des erfindungsgemäßen Verfahrens ist in Figur 2 dargestellt. Hierbei werden die einzelnen Verfahrensschritte wie in der zuvor beschriebenen Ausführungsform in Figur 1 durchgeführt, allerdings parallel mit drei verschiedenen Suspensionen (1). Diese enthalten jeweils eine andere Spezies von markierten Testbakteriophagen (2), welche jeweils spezifisch eine unterschiedliche nachzuweisende Bakterienart bindet, sowie einen Referenz-Bakteriophagen (6). Bei den Markierungen handelt es sich jeweils um fluoreszente Reportermoleküle, welche vorzugsweise jeweils disjunkt sind oder, bei Zuschaltung spektrophotometrischer Detektion mit nachgeschalteter Entmischung, auch teilweise überlappend sein können. Die zu testende Probe (3) wird in dieser bevorzugten Ausführungsform durch Mikrofluidik-Kanäle (25) eines Mikrofluidik-Systems (24) auf die drei Suspensionen (1) verteilt, so dass die Zugabe gleichzeitig erfolgt. Aufgrund der bevorzugt disjunkten oder teilweise überlappenden Markierungen ist folglich ein jeweils spezifischer Nachweis von drei nachzuweisenden Bakterienarten in einer Probe (3) gleichzeitig möglich.

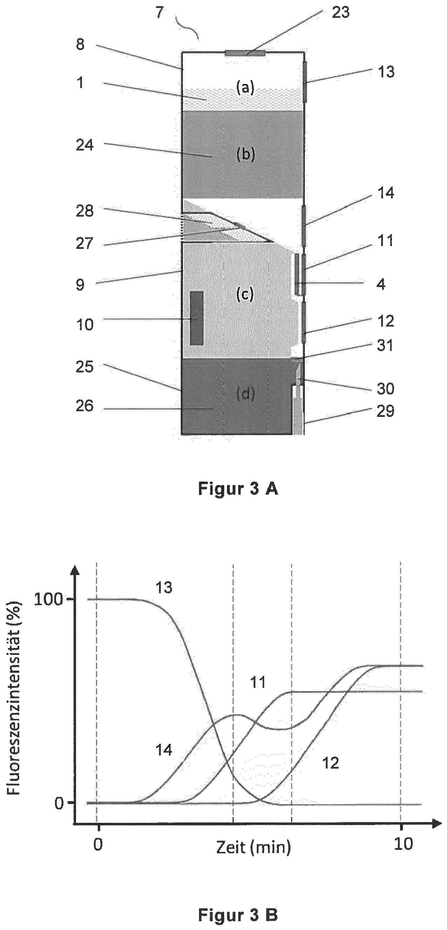

In Figur 3 A ist eine bevorzugte Ausführungsform des erfindungsgemäßen Reaktionsgefäßes (7) dargestellt. Dieses umfasst zunächst vier Kompartimente (a)-(d). Kompartiment (a) ist ein Phagenreservoir (8), welches eine Suspension (1) enthält, die mindestens eine Spezies von markierten Testbakteriophagen umfasst, welche spezifisch an eine nachzuweisende Bakterienart bindet, wobei die Markierung vorzugsweise für die jeweilige Spezies von Testbakteriophagen kennzeichnend ist. Ferner kann die Suspension (1) im Phagenreservoir (8) einen markierten Referenz-Bakteriophagen, welcher an keine nachzuweisende Bakterienart bindet und eine von den Testbakteriophagen disjunkte Markierung aufweist, enthalten. Das Phagenreservoir (8) ist dabei durch ein Septum (23) verschlossen. Kompartiment (b) enthält eine Gelmatrix (24) mit einer Porengröße von 0,01-5 µm als Vorfilter, welche eine Vortrennung des Reaktionsgemisches nach dem Prinzip der Gelfiltration ermöglicht. Dabei werden Bakterien-Bakteriophagen-Komplexe und nicht nachzuweisende Bakterien von ungebundenen Bakteriophagen aufgrund ihres Größenunterschiedes separiert, wobei die größeren Komplexe die Gelmatrix (24) vor den kleineren, ungebundenen Bakteriophagen verlassen. Kompartiment (c) ist ein Auffangreservoir (9), welches für die Aufnahme des Filtrats der Reaktionslösung nach Zugabe einer Probe zu der Suspension (1) und anschließender Filtration vorgesehen ist. Kompartiment (d) stellt ein Abfallreservoir (25) dar, das ein Absorptionsmittel (26) enthält, welches das Filtrat aus dem Auffangreservoir (9) absorbiert. Die Kompartimente (a) und (b) sind durch einen Filter (4) mit einer Porengröße von 0,1 µm bis 0,5 µm von den Kompartimenten (c) und (d) getrennt. In der dargestellten Ausführungsform verfügt das Reaktionsgefäß weiterhin über einen RFID-Chip (10) zur Identifikation sowie über ein zusätzliches, durch ein Septum (27) von den anderen Kompartimenten getrenntes Kompartiment, welches eine Indikatorlösung (28) mit markierten Mikro- oder Nanopartikeln enthält. Diese binden nicht an eine nachzuweisende Bakterienart und passieren den Filter (4) aufgrund ihrer geringen Größe. Daher kann die Indikatorlösung (28) mit den markierten Mikro- oder Nanopartikeln als Alternative zu einem Referenz-Bakteriophagen verwendet werden und mittels Durchstechen der Membran (27) zu der Reaktionslösung geben werden. Das Reaktionsgefäß verengt sich in dieser Ausführungsform zum Filter (4) hin, so dass eine Konzentrierung der Bakterien-Bakteriophagen-Komplexe und der ungebundenen Bakteriophagen vor der Filtration ermöglicht wird. Weiterhin umfasst das dargestellte Reaktionsgefäß (7) insgesamt vier Messfenster (11-14). Dabei ist ein Messfenster (11) im Bereich der Filteroberfläche, welche dem Phagenreservoir zugewandt ist, angeordnet. Ein weiteres Messfenster (12) ist im Bereich des Auffangreservoirs angeordnet. Dadurch wird die Detektion von auf der Filteroberfläche im Retentat immobilisierten Bakterien-Bakteriophagen-Komplexen und von ungebundenen Bakteriophagen im Filtrat ermöglicht. Ein weiteres Messfenster (13) ist im Bereich des Phagenreservoirs angeordnet, so dass die Gesamtheit der Signale, welche von den Markierungen der Bakteriophagen stammen, als Ausgangsreferenz vor Zugabe einer zu testenden Probe detektiert werden kann. Darüber hinaus ist ein viertes Messfenster (14) unterhalb der Gelmatrix angeordnet zur Detektion von Bakterien-Bakteriophagen-Komplexen und ungebundenen Bakteriophagen im Reaktionsgemisch bei Austritt aus der Gelmatrix (24) vor der Filtration. In der gezeigten Ausführungsform weist das Reaktionsgefäß (7) ferner im Bereich des Abfallreservoirs (25) einen integrierten Belüftungsmechanismus (29) bestehend aus Belüftungsnadel (30) und Septum (31) auf. Bei Durchstechen des Septums (31) wird das Reaktionsgefäß belüftet, wodurch die gravimetrische Filtration gestartet wird.

Figur 3 B zeigt den theoretischen Verlauf einer zeitaufgelösten Messung der Fluoreszenz in einer bevorzugten Ausführungsform des erfindungsgemäßen Verfahrens unter Verwendung des erfindungsgemäßen Reaktionsgefäßes (7) aus Figur 3 A. Hierbei wird eine Probe, die auf das Vorliegen einer nachzuweisenden Bakterienart getestet wird, durch das Septum (23) in die Suspension (1) im Phagenreservoir (8) injiziert, durch mehrfaches (3-5x) Schwenken mit der Suspension (1) vermischt und 5-10 min bei Raumtemperatur (RT) inkubiert. Die mindestens eine Spezies von Testbakteriophagen in der Suspension (1) weist dabei fluoreszente Reportermoleküle als Markierung auf. Mit dem Durchstechen des Septums (31) im integrierten Belüftungsmechanismus (29) wird gleichzeitig eine zeitaufgelöste Messung der Fluoreszenzintensität an den vier Messfenstern (11-14) des Reaktionsgefäßes (7) gestartet. Infolge der Belüftung werden die Kompartimente (b) und (c) nacheinander vom Reaktionsgemisch durchströmt, wodurch die Fluoreszenz an Messfenster 13 in Kompartiment (a) im zeitlichen Verlauf immer weiter abnimmt, bis diese nicht mehr detektierbar ist. In der Gelmatrix (24) in Kompartiment (b) werden ungebundene Phagen von größeren Bakterien-Bakteriophagen-Komplexen aufgrund ihres Größenunterschiedes separiert. Die größeren Bakterien-Bakteriophagen-Komplexe verlassen die Gelmatrix (24) vor den kleineren ungebundenen Bakteriophagen, wodurch die Fluoreszenz an Messfester (14) zunächst zunimmt bis alle Bakterien-Bakteriophagen-Komplexe diesen Messbereich passiert haben. Danach nimmt die Fluoreszenz wieder etwas ab, bis schließlich die ungebundenen Bakteriophagen die Gelmatrix verlassen, was durch einen erneuten Anstieg der Fluoreszenz an Messfenster (14) gekennzeichnet ist. Auf dem Filter (4) werden die Bakterien-Bakteriophagen-Komplexe immobilisiert, so dass deren Fluoreszenzsignal an Messfenster (11) zunimmt, bis alle Bakterien-Bakteriophagen-Komplexe bzw. nicht nachzuweisenden Bakterienarten auf dem Filter (4) immobilisiert sind. Ungebundene Bakteriophagen passieren den Filter (4) und treten als ansteigendes Fluoreszenzsignal in Messfenster (12) im Auffangreservoir (9) in Erscheinung. Überschüssiges Reaktionsgemisch, das das Reaktionsgefäß vollständig durchlaufen hat, wird in Kompartiment (d) gesammelt und durch ein Absorptionsmittel (26) gebunden.

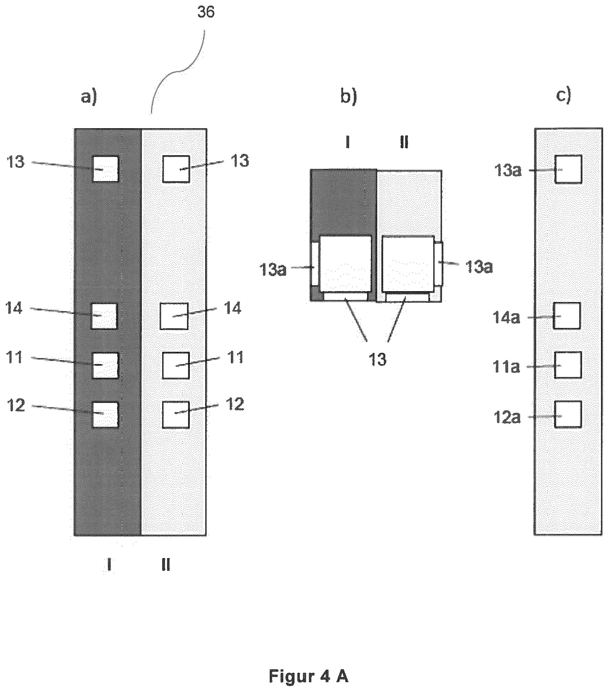

Darüber hinaus zeigt Figur 4 A die schematische Darstellung einer bevorzugten Ausführungsform der erfindungsgemäßen Kartusche (36) zum Nachweis von Bakterien in a) Vorderansicht, b) Aufsicht und c) Seitenansicht. Die erfindungsgemäße Kartusche (36) umfasst zwei parallel angeordnete Reaktionsgefäße (I, II), welche jeweils der in Figur 3 A dargestellten Ausführungsform des erfindungsgemäßen Reaktionsgefäßes (7) im Wesentlichen entsprechen. So enthalten die parallelen Reaktionsgefäße (I, II) jeweils vier Messfenster (11-14). Ein Messfenster (11) ist im Bereich der Filteroberfläche, welche dem Phagenreservoir zugewandt ist, angeordnet. Ein weiteres Messfenster (12) ist im Bereich des Auffangreservoirs angeordnet. Dadurch wird die Detektion von auf der Filteroberfläche im Retentat immobilisierten Bakterien-Bakteriophagen-Komplexen und von ungebundenen Bakteriophagen im Filtrat ermöglicht. Ein weiteres Messfenster (13) ist im Bereich des Phagenreservoirs angeordnet, so dass die Gesamtheit der Signale, welche von den Markierungen der Bakteriophagen stammen, als Ausgangsreferenz vor Zugabe einer zu testenden Probe detektiert werden kann. Darüber hinaus ist ein viertes Messfenster (14) unterhalb der Gelmatrix angeordnet zur Detektion von Bakterien-Bakteriophagen-Komplexen und ungebundenen Bakteriophagen im Reaktionsgemisch bei Austritt aus der Gelmatrix (24) vor der Filtration. In der gezeigten Ausführungsform weisen die parallelen Reaktionsgefäße (I, II) weiterhin jeweils vier Anregungsfenster (11a-14a) im Bereich der Messfenster (11-14) auf, welche der Anregung von Reportermolekülen mit fluoreszenten Eigenschaften dienen.

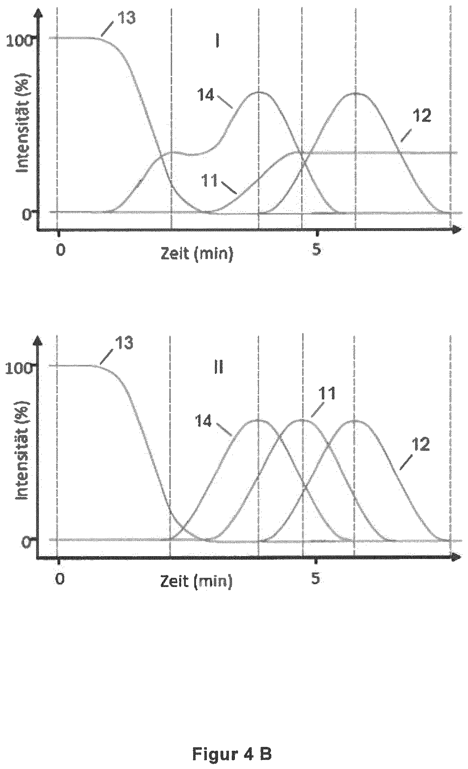

Figur 4 B zeigt die theoretischen Verläufe einer zeitaufgelösten Messung der Fluoreszenz in einer bevorzugten Ausführungsform des erfindungsgemäßen Verfahrens unter Verwendung der erfindungsgemäßen Kartusche (7) aus Figur 4 A. Hierbei wird eine Probe, die auf das Vorliegen einer nachzuweisenden Bakterienart getestet wird, in das Reaktionsgefäß (I) injiziert. In das Reaktionsgefäß (II) wird lediglich Neutrallösung ohne nachzuweisende Bakterienart injiziert. In beiden Reaktionsgefäßen (I und II) wird mit Neutrallösung gespült, so dass sich keine Bakterien-Bakteriophagen-Komplexe bilden können. Wie bereits für den Verlauf in Figur 3 B im Detail beschrieben, nimmt die Fluoreszenz an Messfenster 13 in beiden Reaktionsgefäßen (I und II) im zeitlichen Verlauf der Filtration immer weiter ab, bis diese nicht mehr detektierbar ist. Im Fall des Reaktionsgefäßes (I) verlassen die größeren Bakterien-Bakteriophagen-Komplexe aufgrund der Vortrennung durch Gelfiltration die Gelmatrix vor den kleineren ungebundenen Bakteriophagen, wodurch die Fluoreszenz an Messfester (14) zunächst zunimmt bis alle Bakterien-Bakteriophagen-Komplexe diesen Messbereich passiert haben. Danach nimmt die Fluoreszenz wieder etwas ab, bis schließlich die ungebundenen Bakteriophagen die Gelmatrix verlassen, was durch einen erneuten Anstieg der Fluoreszenz an Messfenster (14) gekennzeichnet ist. Da sich im Reaktionsgefäß (II) wegen der fehlenden Probenzugabe keine Bakterien-Bakteriophagen-Komplexe bilden können, ist der Anstieg der Fluoreszenz an Messfenster (14) allein auf ungebundene Testbakteriophagen zurückzuführen, eine "Schulter" in der Intensität der Fluoreszenz an Messfenster (14) ist daher nicht zu beobachten. Im Zuge des Spülens mit Neutrallösung werden die ungebundenen Bakteriophagen (im Fall von Reaktionsgefäß (I) auch eventuell verbliebene Bakterien-Bakteriophagen-Komplexe) nach und nach jeweils aus diesem Messbereich entfernt, was jeweils in einer Abnahme der Fluoreszenz an Messfenster (14) gegen Null resultiert. Die in Reaktionsgefäß (I) gebildeten Bakterien-Bakteriophagen-Komplexe werden auf dem Filter immobilisiert, so dass das Fluoreszenzsignal an Messfenster (11) zunimmt, bis sämtliche Bakterien-Bakteriophagen-Komplexe auf dem Filter immobilisiert sind. Danach ist keine Zunahme der Fluoreszenz an Messfenster (11) des Reaktionsgefäßes mehr zu verzeichnen, vielmehr bleibt die Fluoreszenz in diesem Messbereich auch während des Spülens mit Neutrallösung konstant. Demgegenüber nimmt das Fluoreszenzsignal an Messfenster (11) des Reaktionsgefäßes (II) zunächst ebenfalls zu, was jedoch auf eine temporäre Ansammlung von Testbakteriophagen im Bereich des Filters zurückzuführen ist. Durch Spülen mit Neutrallösung passieren jedoch sämtliche Testbakteriophagen den Filter, so dass die Fluoreszenz in diesem Messbereich abnimmt und die Fluoreszenzintensität in Messfenster (12) im Bereich des nachfolgenden Auffangreservoirs des Reaktionsgefäßes (II) zunimmt. Durch Spülen mit Neutrallösung werden die Testbakteriophagen schließlich in das Abfallreservoir überführt, wodurch das Fluoreszenzsignal in Messfenster (12) des Reaktionesgefäßes (II) wieder abnimmt. Ein ähnlicher Verlauf des Fluoreszenzsignals an Messfenster (12) ist auch im Reaktionsgefäß (I) zu beobachten, allerdings stammt die Fluoreszenz hierbei nur von den verbliebenen, ungebundenen Bakteriophagen,

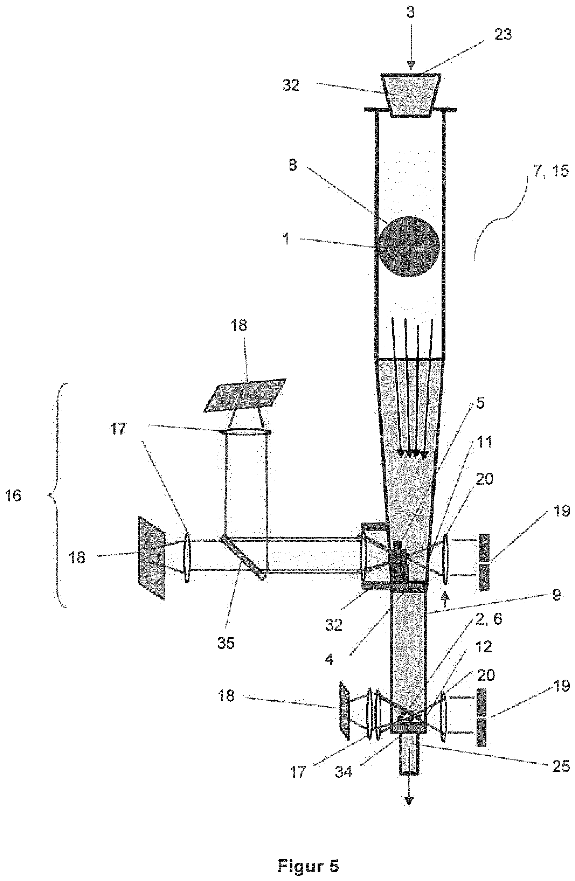

Eine schematische Darstellung des Aufbaus von erfindungsgemäßem Reaktionsgefäß (7) und Messgerät (15) in einer weiteren bevorzugten Ausführungsform ist in Figur 5 dargestellt. Das gezeigte Reaktionsgefäß (7) enthält ein Phagenreservoir (8) mit eindrückbaren Membranen, welches wiederum eine Suspension (1) enthält, die mindestens eine Spezies von markierten Testbakteriophagen .(2) umfasst, welche spezifisch an eine nachzuweisende Bakterienart bindet, wobei die Markierung für die jeweilige Spezies von Testbakteriophagen (2) vorzugsweise kennzeichnend ist. Ferner kann die Suspension (1) im eindrückbaren Phagenreservoir (8) einen markierten Referenz-Bakteriophagen (6) enthalten, welcher an keine nachzuweisende Bakterienart bindet und eine von den Testbakteriophagen (2) disjunkte Markierung aufweist, enthalten. Das Reaktionsgefäß (7) ist im oberen Bereich mit einem Septum (23) verschlossen, an das sich ein Grobfilter (32) mit einer Porengröße von 0,5-10,0 µm anschließt, um größere Schwebteile und etwaige Verunreinigungen zu entfernen. Dabei kann auch ein mehrstufiger Grobfilter (32) mit abnehmenden Porengrößen von 0,5-10 µm verwendet werden. Durch Injektion einer zu testenden Probe (3) in die Suspension (1) im Phagenreservoir (8) werden die Membranen des Phagenreservoirs (8) eingedrückt, so dass die entstehende Reaktionslösung beginnt, aufgrund der Gravitation in Richtung des Filters (4) zu fließen, welcher eine Porengröße von 0,2 µm aufweist. In der gezeigten Ausführungsform verengt sich das Reaktionsgefäß (7) nach unten hin, so dass eine Konzentrierung der Bakterien-Bakteriophagen-Komplexe (5) und der ungebundenen Bakteriophagen (2, 6) vor der Filtration ermöglicht wird. Zusätzlich erfolgt eine Konzentrierung elektromagnetisch durch selektives Zu- und Abschalten eines homogenen Magnetfeldes. Hierfür umfasst das Messgerät (15) eine Ringspule (33) als Elektromagneten, welche im Bereich der Filteroberfläche des Filters (4) angeordnet ist. Darüber hinaus sind die Testbakteriophagen (2) der mindestens einen Spezies sowie der optionale Referenzphage (6) mit magnetischen Beads beschichtet. An den Filter 4 schließt sich ein Auffangreservoir (9) zur Aufnahme des Filtrats nach erfolgter Filtration an. An das Auffangreservoir (9) schließt sich wiederum ein weiteres Kompartiment an, welches als Abfallreservoir (25) bezeichnet wird und durch einen Endfilter (34) mit einer Porengröße von ≤ 0,05 µm von dem Auffangreservoir (9) getrennt ist. Durch den Endfilter (34) werden sämtliche Bakteriophagen entfernt, so dass das verbleibende Filtrat kostengünstig und gefahrlos entsorgt werden kann. In dieser Ausführungsform besteht die Markierung der Bakteriophagen (2, 6) ebenfalls aus mindestens einem Reportermolekül mit fluoreszenten Eigenschaften. Dementsprechend umfasst das Messgerät (15) neben einem Schacht, in den das Reaktionsgefäß (7) eingeschoben wird, zwei Detektions-Optiken (16), welche jeweils im Bereich der Messfenster (11, 12) des eingeschobenen Reaktionsgefäßes (7) angeordnet sind. In der vorliegenden Ausführungsform bedeutet dies, dass eine Detektions-Optik (16) im Messbereich an der Filteroberfläche des Filters (4) zur Detektion von Bakterien-Bakteriophagen-Komplexen (5) angeordnet ist, während die zweite Detektions-Optik (16) im Messbereich an der Filteroberfläche des Endfilters (34) zur Detektion von ungebundenen Bakteriophagen (2, 6) angeordnet ist. Die Detektions-Optiken (16) enthalten jeweils eine LED (19) als Beleuchtungseinheit, welche Licht im Wellenlängenbereich von 200-1000 nm, vorzugsweise 350-700 nm, emittiert, je nach den Absorptionseigenschaften der verwendeten fluoreszenten Reportermoleküle. Dabei soll möglichst eine maximale Absorption erzielt werden, um ein entsprechend starkes Fluoreszenzsignal zu generieren. Ferner enthalten die Detektions-Optiken (16) jeweils ein Linsensystem (20) zur Fokussierung des von der LED emittierten Lichts auf die oben beschriebenen Messbereiche und jeweils ein weiteres Linsensystem (17) zur Fokussierung des emittierten Lichts der fluoreszenten Reportermoleküle auf mindestens einen Sensor (18), wie z.B. einem Photomultiplier, einem CCD-Sensor oder einem CMOS-Sensor als optischen Sensor. Alternativ oder zusätzlich kann ein Spektral-Photometer (18) als Sensor zur Aufzeichnung des Emissions-Spektrums verwendet werden. Ein Spektral-Photometer wird hierbei nicht als optischer Sensor im strengeren Sinne verstanden. Die Sensoren (18) der beiden Detektions-Optiken (16) detektieren dabei jeweils das einfallende Licht und leiten die erhaltenen Detektionssignale an einen Prozessor weiter, welcher jeweils mit diesen verbunden ist. Der Prozessor verarbeitet schließlich die erhaltenen Detektionssignale und leitet die Detektionsergebnisse an ein mit dem Prozessor verbundenes Display weiter, welche diese optisch an einen Benutzer ausgibt (nicht gezeigt). Darüber hinaus kann eine oder können beide Detektionsoptiken (16) einen Strahlenteiler (35) zur spektralen Trennung mehrerer Farbkanäle enthalten.

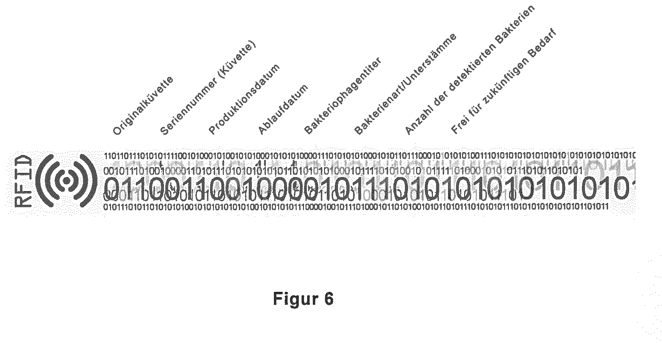

In Figur 6 ist ein RFID Chip als eine Ausführungsform des Mittels zur Identifizierung des erfindungsgemäßen Reaktionsgefäßes dargestellt, welcher unter anderem Daten hinsichtlich der Seriennummer, des Produktionsdatums, des Ablaufdatums, des Phagentiters in der Suspension und/oder der nachzuweisenden Bakterienart(en) bzw. der nachzuweisenden Stämme enthält. Das RFID Chip ermöglicht eine einfache und eindeutige Identifizierung des Reaktionsgefäßes, wodurch die Gefahr von Verwechslungen von Reaktionsgefäßen minimiert und somit die Verlässlichkeit eines damit durchgeführten Nachweisverfahrens erhöht wird.

### Experimenteller Teil

### 1. Isolierung von Bakteriophagen

Da Bakteriophagen keinen eigenen Stoffwechsel besitzen, können sie nur auf einem geeigneten Wirtsbakterium angezogen und vermehrt werden. Die Wirtsbakterien benötigen ein Nährmedium, in dem sie optimal wachsen können. Je besser das Wachstum der Bakterien desto höher ist die Ausbeute an Bakteriophagen. In einem ersten Schritt wird pulverförmiges Nährmedium in Wasser gelöst und in einem Autoklaven sterilisiert. Danach wird das sterile Nährmedium direkt aus dem Autoklaven in einen Fermenter gepumpt und dort auf die optimale Temperatur für das Wachstum der Wirtsbakterien vorgeheizt (z.B. 30°C). Sobald die Nährlösung die Betriebstemperatur erreicht hat, werden aus einer Bakterienvorkultur Wirtsbakterien in den Fermenter gegeben. Diese Bakterien beginnen nun, sich zu teilen. Nach einer kurzen Adaptationsperiode wachsen die Wirtsbakterien exponentiell, d.h. mit der höchstmöglichen, für den Bakterientyp spezifischen, Wachstumsrate.

Das Wachstum der Bakterienzellen wird über die Messung der optischen Dichte bei einer Wellenlänge von 600 nm (OD₆₀₀) in einem Photometer verfolgt. Wenn eine in Vorexperimenten optimierte Zelldichte erreicht ist, z.B. 5 x 10⁸ Bakterien pro Milliliter Nährlösung, werden aus einer Bakteriophagen-Vorkultur Bakteriophagen zum Fermenter zugegeben. Die Konzentration der Bakteriophagen in der Vorkultur wird im Vorfeld mittels der klassischen Titrationsmethode, einer biologischen Aktivitätsnachweis-methode, bestimmt (siehe z.B. Martha R. J. Clokie, Andrew M. Kropinski (eds.), Bacteriophages: Methods and Protocols, Volume 1: Isolation, Characterization, and Interactions, vol. 501, C 2009 Humana Press, a part of Springer Science+Business Media). Die Konzentration wird so eingestellt, dass idealerweise jedes Bakterium im Fermenter durch mindestens einen Bakteriophagen erkannt und infiziert wird. Nach erfolgreicher Infektion produzieren die Wirtsbakterien nun neue Bakteriophagen anstatt wie bisher neue Bakterien. Der Vermehrungsprozess der Bakteriophagen dauert etwa eine Stunde, und in dieser Zeit werden pro Bakterienzelle etwa 100 neue Bakteriophagen produziert.

Am Ende des Prozesses platzen die Bakterien auf und entlassen die neuen Bakteriophagen ins Nährmedium. Die so im Fermenter entstandene Lösung ist hoch viskos, da sie neben den neuen Bakteriophagen auch alle Komponenten der aufgeplatzten Bakterienzelle (Bruchstücke der Zellwand, Lipide, Ribosomen, Erbmaterial der Bakterien etc.) und auch die ursprünglichen Komponenten des Nährmediums (Zucker, Salze und Proteine) enthält. Deshalb müssen die Bakteriophagen erst über ein mehrstufiges Filtrationsverfahren aufgereinigt werden, bevor sie für eine Markierungsreaktion zur Verfügung stehen. Nach der Aufreinigung erfolgt eine Sterilfiltration, z.B. über 0,2 µm Sartobran® Filterkartuschen von Sartorius Stedim Biotech, kombiniert mit der Abfüllung in Vorratsbehälter (Kegs).

In dieser Form sind Bakteriophagen stabil, lassen sich einfach transportieren und über Monate ohne Aktivitätsverlust lagern.

### 2. Markierung von Bakteriophagen mit Reportermolekülen

Um Nebenreaktionen während der Kopplung mit Reportermolekülen und schlechte Kopplungseffizienzen zu vermeiden, werden die Phagen vor der Markierung mit den aktivierten Reporter-Molekülen gereinigt und umgepuffert. Während der Isolierung von Proteinen muss in vielen Fällen ein Pufferaustausch vorgenommen werden, z.B. wenn mehrere chromatographische Verfahren mit unterschiedlichen Puffern hintereinander verwendet werden sollen.

Die für die Markierungsexperimente eingesetzten Phagen werden mittels der folgenden Standardverfahren gereinigt und gegebenenfalls konzentriert:
- durch Ultrafiltration, wobei der Größenausschlußbereich (molecular weight cut-off (MWCO)) > 100 kDa sein sollte:
- durch Polyethylenglycol/Natriumchlorid-Präzipitation
- durch Größenauschluß-Chromatographie (Gelfiltration)
- durch lonenaustausch-Chromatographie
- durch Dialyse

Die Trennung kleiner, überschüssiger Moleküle, wie Salze, Farbstoffe oder Biotin, aus Lösungen von Phagen wird durch Fällung mit Polyethylenglycol und Natriumchlorid und anschließender Zentrifugation durchgeführt (Jaye, D.L., Edens, H.A., Mazzucchelli, L., Parkos, C.A., 2001. Novel G protein-coupled responses in leukocytes elicited by a chemotactic bacteriophage displaying a cell type-selective binding peptide. J. Immunol. 166, 7250).

Im Anschluß an die Inkubation der Kopplungsreaktion werden die Phagen-Konjugate mindestens zweimal mit Polyethylenglycol 8000 (PEG 8000) und einer NaCI-Lösung gefällt. Dieses minimiert den Anteil freier Reporter-Moleküle in der Konjugatlösung. Abschließend wird das Konjugatpellet in einem geeigneten Puffer resuspendiert. Gelfiltrationssäulen mit einem Größenausschlussmaterial auf Dextran-Basis mit einem MWCO > 25 kDa können ebenfalls eingesetzt werden. Die lonenaustausch-Chromatographiebasierte Reinigung gestattet ebenfalls die rasche Trennung der Konjugate von den kleinen Molekülen, wobei der geeignetste Gradient und die Art sowie die Stärke des lonenaustauschermaterials von den eingesetzten Markermolekülen und der Natur der spezifischen Phagen abhängen. Eine Optimierung der Parameter ist vorab notwendig.

### Beispiel 1:

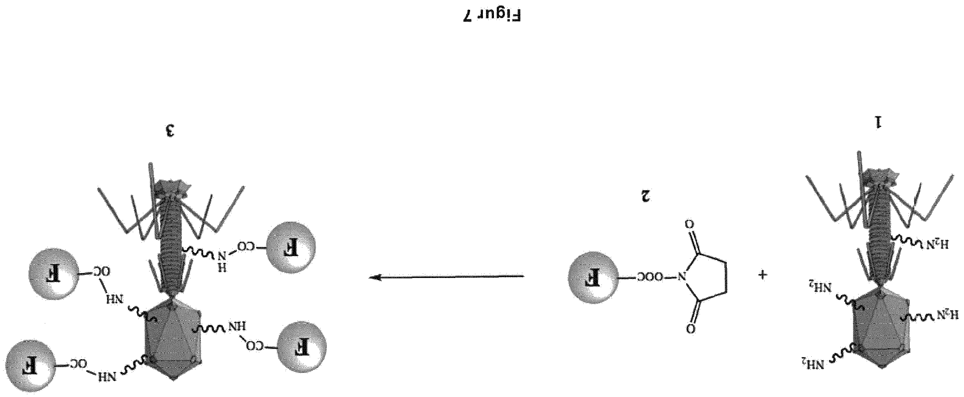

1x10¹¹ pfu von zuvor gereinigten und konzentrierten TB54 Bakteriophagen (siehe oben) wurden in 10 ml PBS mit 0,1 M NaHCO₃ (pH 8,5) resuspendiert. Daraufhin 100 nmol 5(6)-Carboxyfluorescein-NHS-Ester pro 1x10¹⁰ pfu zugegeben. Die Kopplungsreaktion verläuft bei Raumtemperatur im Dunkeln für eine Stunde. Wie in Figur 7 schematisch dargestellt reagiert der NHS-Ester von 5(6)-Carboxyfluorescein (2) mit den primären Aminogruppen von Proteinen auf der Oberfläche der TB54 Phagen (1) unter Ausbildung stabiler Amidbindungen.

Die Reinigung der Konjugate erfolgt entweder per Präzipitation oder mittels chromatographischer Methoden wie oben beschrieben. Anschließend wird das Konjugat photophysikalisch mittels UV/vis- und Fluoreszenzspektroskopie charakterisiert.

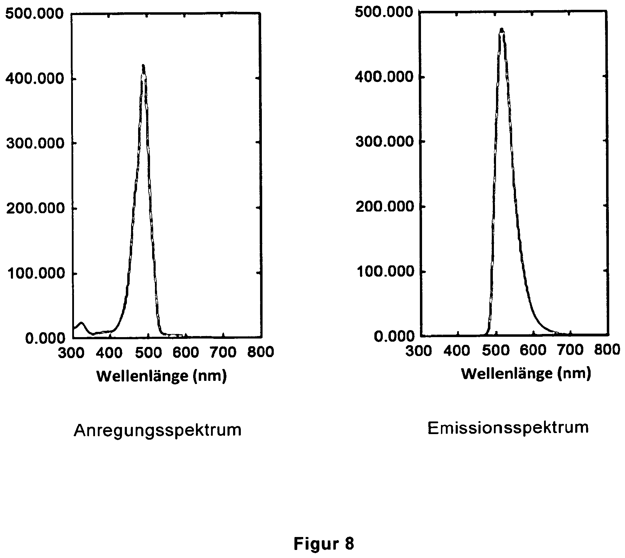

In Figur 8 sind die Anregungs- und Emissionsspektren von 5(6)-Carboxyfluorescein-markierten TB54 Bakteriophagen gezeigt. Die Spektren lassen das Anregungs- bzw. Emissionsmaximum von 5(6)-Carboxyfluorescein bei einer Wellenlänge von 495 nm bzw. im Bereich von 520-530 nm erkennen. Diese Wellenlängen wurden im Folgenden auch für fluoreszenzmikroskopische Untersuchungen der Bakterien-Bakteriophagen-Interaktion verwendet.

### Beispiel 2:

Eine Fluorescein-isothiocyanat (FTIC)-Lösung in 0,1 M Carbonat-Puffer, pH 9,0 wird vorbereitet. Ein entsprechendes Aliquot wird zu der, wie in Beispiel 1 vorbereiteten Phagen-Lösung gegeben und vorsichtig durchmischt. Die Phagensuspension wird für eine Stunde bei Raumtemperatur im Dunkeln inkubiert. Die Reinigung und Charakterisierung des Konjugates erfolgt wie in Beispiel 1 beschrieben.

### 3. Spezifität der Bakterien-Bakteriophagen-Interaktion

Um die enorme Wirtsspezifität einer markierten Spezies von Testbakteriophagen zu demonstrieren, wurden in einem vorbereitenden Schritt Übernachtkulturen von *Escherichia* coli-Bakterien der Stämme C600 (PTC Phage Technology Center GmbH), ECOR34 (PTC Phage Technology Center GmbH) und XL10-Gold (Agilent) unter Standardbedingungen bei 37°C in 10 ml TSB *(Tryptic* Soy *Broth*)(Sigma-Aldrich) hergestellt. Die Übernachtkulturen wurden am folgenden Tag verdünnt (1:10 in TSB) und für weitere zwei Stunden bei 37°C inkubiert. Die auf diese Weise erhaltenen Bakteriensuspensionen wurden anschließend zu gleichen Teilen vermischt (1:1:1). Für den fluoreszenzmikroskopischen Nachweis der Bakteriophagen-Wirt Interaktion wurden 10 µl der Bakterien-Mischkultur E. *coli* Bakterien (C600, ECOR34, XL10-Gold) in 990 µl einer Suspension von 5(6)-Carboxyfluorescin-markierten TB54 Bakteriophagen in PBS, pH 7,4 gegeben (Bakterientiter: ∼1x10⁶ Zellen/ml; Phagentiter: ∼1x10⁸ pfu/ml) und für 5 min bei Raumtemperatur (RT) inkubiert. Von dem Reaktionsgemisch wurden anschließend 20 µl auf einen Objektträger gegeben und fluoreszenzmikroskopisch untersucht (Eclipse *Ti,* 100x Objektiv, Nikon).

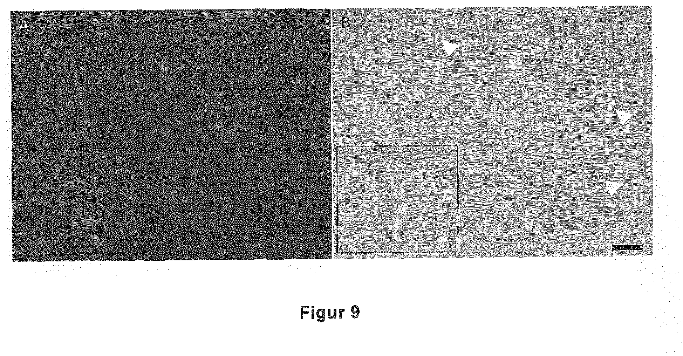

Figur 9 A zeigt die erhaltene fluoreszenzmikroskopische Aufnahme nach Inkubation der E. *coli* Bakterienstämme C600, ECOR34 und XL10-Gold mit 5(6)-Carboxyfluorescin-markierten TB54 Bakteriophagen. Der Bildausschnitt in der linken unteren Ecke zeigt eine vergrößerte Darstellung des weiß umgrenzten Bildbereichs.

In Figur 9 B ist eine entsprechende Hellfeldaufnahme nach Inkubation der *E. coli* Bakterienstämme C600, ECOR34 und XL10-Gold mit 5(6)-Carboxyfluorescin-markierten TB54 Bakteriophagen dargestellt. Der Bildausschnitt in der linken unteren Ecke zeigt eine vergrößerte Darstellung des weiß umgrenzten Bildbereichs. Die weißen Pfeile weisen dabei auf nichtmarkierte Bakterien hin, die sich morphologisch von den, mit markiertenTB54-Phagen infizierten E. *coli* C600 Bakterienzellen unterscheiden und den Stämmen ECOR34 bzw. XL10-Gold zuzuordnen sind (Größenmarker: 10 µm).

Die Versuchsergebnisse belegen die enorme Spezifität der mit 5(6)-Carboxyfluorescin-markierten TB54 Bakteriophagen. Diese binden ausschließlich an den E. *coli* C600 Bakterienstamm, während die Stämme ECOR34 und XL10-Gold nicht infiziert werden. Daher weisen im Reaktionsgemisch nur Bakterien des E. *coli* C600 Stamms durch Komplexbildung Fluoreszenzsignale auf. Somit bietet das erfindungsgemäße Verfahren, aufgrund der enormen Wirtsspezifität von Bakteriophagen, einen eindeutigen und verlässlichen Nachweis von Bakterienarten. Dabei können sogar einzelne Stämme innerhalb einer Bakterienart nachgewiesen werden.

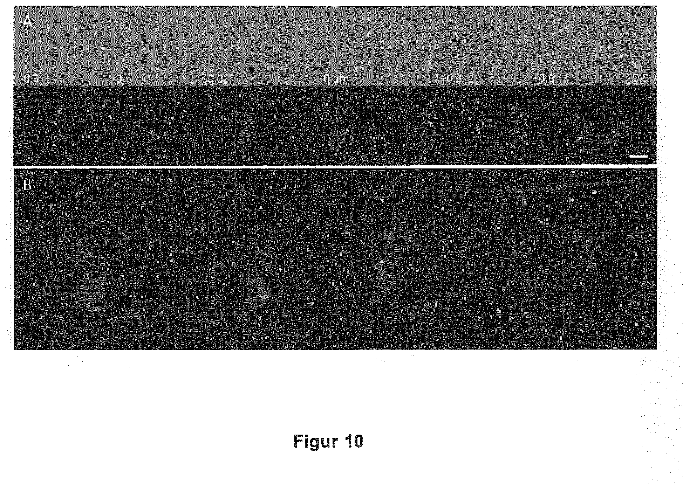

Figur 10 A zeigt darüber hinaus optische Schnittbilder der in diesem Versuch erhaltenen E. *coli* C600-TB54 Bakteriophagen-Komplexe. Die optischen Schnitte wurden Abständen von 0,3 µm angefertigt (Größenmarker 2 µm). In der oberen Reihe sind die Komplexe im Durchlicht dargestellt, die untere Reihe zeigt dieselben Komplexe in fluoreszenzmikroskopischer Darstellung.

Die Verteilung der punktförmigen Fluoreszenzsignale lässt dabei eine Lokalisation der fluoreszenten Reportermoleküle (5(6)-Carboxyfluorescin) auf der Bakterienoberfläche erkennen.

In Figur 10 B sind dreidimensionale Rekonstruktionen der erhaltenen E. *coli* C600-TB54 Bakteriophagen-Komplexe in unterschiedlichen perspektivischen Ansichten dargestellt, was die Oberflächenlokalisation der der fluoreszenten Reportermoleküle bestätigt.

### 4. Sensitivität des Verfahrens

Zur Ermittlung der Sensitivität des Nachweisverfahrens für Bakterien wurde durch serielle Verdünnung einer Ausgangssuspension von 5(6)-Carboxyfluorescin-markierten TB54 Bakteriophagen (PBS, pH 7,4, Phagentiter: ∼1x10⁸ pfu/ml) mit PBS, pH 7,4 eine Verdünnungsreihe erstellt. Von den jeweiligen Verdünnungsstufen mit ∼1x10⁷ pfu/ml, ∼1x10⁶ pfu/ml, ∼1x10⁵ pfu/ml, ∼1x10⁴ pfu/ml und ∼1x10³ pfu/ml wurden jeweils 1 ml in eine Messküvette gegeben und fluoreszenzspektroskopisch untersucht (Qwave compact USB Spektrometer, RGB Lasersystems). Als Referenz wurde 1 ml PBS Puffer ohne markierte TB54 Bakteriophagen verwendet.

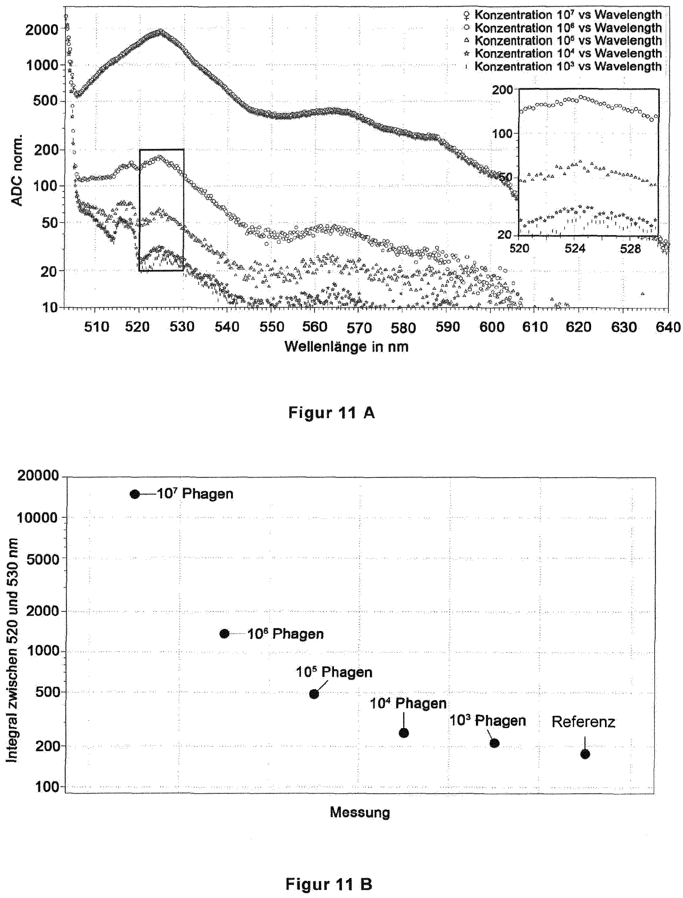

Die erhaltenen Fluoreszenzspektren sind in Figur 11 A dargestellt. Für sämtliche Verdünnungsstufen ist jeweils ein distinktes Maximum der Emission im Wellenlängenbereich zwischen 520 und 530 nm zu erkennen, was dem Emissionsmaximum von 5(6)-Carboxyfluorescin entspricht. In Figur 11 B ist daher das Integral der Fluoreszenz im Wellenlängenbereich zwischen 520 und 530 nm für die verschiedenen Phagentiter dargestellt. Dabei ist das erhaltene Fluoreszenzintegral für eine Phagenmenge von nur ∼1x10³ pfu noch deutlich von der Referenz unterscheidbar, d.h. es gelingt ein spezifischer Nachweis ab ∼1x10³ Bakteriophagen.

Bei einer durchschnittlichen Menge von ∼1x10² gebundenen Bakteriophagen pro infiziertem Bakterium bedeutet dies, dass durch das erfindungsgemäße Verfahren ein spezifischer Nachweis bereits ab einer Bakterienkonzentration von nur -10 Bakterienzellen/ml erbracht werden kann. Das erfindungsgemäße Verfahren ist damit nicht nur hoch-spezifisch, sondern auch höchst sensitiv.

### 5. Spezifischer Nachweis einer Bakterienart bzw. eines Stamms einer Bakterienart.

Im Folgenden ist der spezifische Nachweis für den Bakterienstamm *Escherichia coli* C600 mit Hilfe des erfindungsgemäßen Verfahrens bei unterschiedlichen Bakterienkonzentrationen beschrieben.

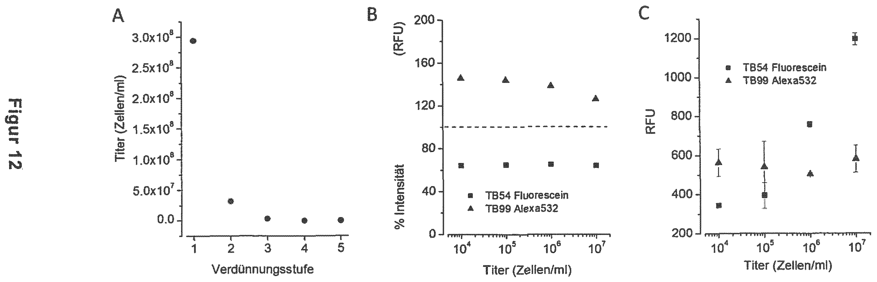

Dazu wurde zunächst eine Übernachtkultur des Bakterienstamms *Escherichia coli* C600 (PTC Phage Technology Center GmbH) unter Standardbedingungen bei 37°C in 10 ml TSB (*Tryptic Soy* Broth)(Sigma-Aldrich) hergestellt. Die Übernachtkultur wurde am folgenden Tag verdünnt (1:10 in TSB) und für weitere zwei Stunden bei 37°C inkubiert. Von der erhaltenen Kultur wurde im Anschluss eine Verdünnungsreihe durch serielle Verdünnung mit TSB erstellt. Zur Bestimmung der Bakterienkonzentration in den seriell verdünnten Bakteriensuspensionen wurde die optische Dichte bei 600 nm (OD₆₀₀) gemessen. Figur 12 A zeigt die jeweils erhaltenen Daten der Bakterientiter für die verschiedenen Verdünnungsstufen.

Die Ausgangsintensität der Fluoreszenz in der Phagensuspension vor Zugabe einer zu testenden Probe wurde bestimmt, indem 900 µl einer Suspension von 5(6)-Carboxyfluorescin-markierten TB54 Bakteriophagen und Alexa532-markierten TB99 Bakteriophagen (∼10⁸ pfu/ml in PBS, versetzt mit 10 mM MgCl₂) mit 100 µl TSB-Medium vermischt und für 5-10 min bei Raumtemperatur (RT) inkubiert wurden. Der Alexa532-markierte TB99 Bakteriophage dient dabei als Referenz-Bakteriophage mit disjunkter Markierung. Die Fluoreszenzsignale des Testbakteriophagen (5(6)-Carboxyfluorescin-TB54) und des Referenz-Bakteriophagen (Alexa532-TB99) wurden anschließend mit einem Plattenlesegerät (Perkin Elmer) gemessen. Die erhaltenen Messwerte wurden als Ausgangsintensität bzw. 100% Signalintensität der Phagensuspension vor Zugabe einer zu testenden Probe definiert, welche im Graph in Figur 12 B als gestrichelte Linie eingezeichnet sind.

Weiterhin wurden jeweils 900 µl der oben genannten Suspension mit jeweils 100 µl der seriell verdünnten E. *coli* C600 Bakteriensuspensionen (∼10⁴-10⁷ Zellen/ml in TSB) vermischt und die Reaktionsgemische ebenfalls für 5-10 min bei Raumtemperatur (RT) inkubiert. Die Reaktionsgemische wurden anschließend jeweils durch einen Filter mit einer Porengröße von 0,4 µm (Micropore Membran, Milipore) filtriert.

Die Fluoreszenz in den erhaltenen Filtraten wurde jeweils mit einem Plattenlesegerät (Perkin Elmer) gemessen. Abbildung 12 B zeigt jeweils die Fluoreszenzintensitäten von ungebundenen 5(6)-Carboxyfluorescin-markierten TB54 Bakteriophagen (Quadrate) und Alexa532-markierten TB99 Bakteriophagen (Dreiecke) im Filtrat. Die angegebenen Fluoreszenzintensitäten wurden jeweils durch Normalisierung auf den ermittelten Ausgangsintensitätswert erhalten, d.h. die gemessenen Fluoreszenzintensitäten wurden jeweils in Bezug zur Ausgangsintensität gesetzt. Dabei ist zu erkennen, dass die Fluoreszenzintensität der Testbakteriophagen (5(6)-Carboxyfluorescin-TB54 als Quadrate) deutlich abnimmt. Dies zeigt, dass die Testbakteriophagen den Bakterienstamm E. *coli* C600 spezifisch infizieren und die entsprechenden Bakterien-Bakteriophagen-Komplexe bilden, welche durch Filtration entfernt werden. Dadurch ist die Anzahl ungebundener Testbakteriophagen im Filtrat geringer als in der anfänglichen Phagensuspension, was sich in der Abnahme der Fluoreszenzintensität im Filtrat wiederspiegelt.

Die Fluoreszenzintensitäten im Retentat sind in Abbildung 12 C dargestellt. Die dargestellten Messwerte wurden jeweils fluoreszenzmikroskopisch, durch Ablichtung der Filteroberflächen mit einem Standardobjektiv (20x, Nikon) und Berechnung der mittleren Bildintensität aus drei Bildern pro Filterfläche ermittelt (Mittelwert ± Standardabweichung, *N*=3). Die Daten zeigen, dass mit Zunahme der Bakterienkonzentration in der zugegebenen Probe, die Fluoreszenzintensität des Testbakteriophagen (5(6)-Carboxyfluorescin-TB54) zunimmt, während sich die Fluoreszenzintensität des Referenz-Bakteriophagen (Alexa532-TB99) kaum ändert. Dies lässt darauf schließen, dass der Testbakteriophage die nachzuweisenden Bakterien spezifisch bindet und mit steigender Konzentration der Bakterien auch die Konzentration der gebildeten Bakterien-Bakteriophagen-Komplexe zunimmt, welche schließlich durch die Filtration auf der Filteroberfläche immobilisiert werden. Der Referenz-Bakteriophage bindet dagegen nicht an die nachzuweisende Bakterienart bzw. den nachzuweisenden Bakterienstamm, so dass die Konzentration der Bakterien keinen Einfluss auf dessen gemessene Fluoreszenzintensität im Retentat hat.

Wie gezeigt, erlaubt das erfindungsgemäße Verfahren einen hoch-spezifischen und hoch-sensitiven Nachweis von Bakterien. Das Verfahren ist dabei einfach durchzuführen und liefert sehr verlässliche Ergebnisse. Zudem ist der Zeitaufwand des Verfahrens gering, so dass ein Nachweis innerhalb von weniger als einer Stunde ab Probenzugabe gelingt.

## Patentansprüche

1. Verfahren zum Nachweis von Bakterien, umfassend die Schritte
A) Bereitstellen einer oder mehrerer Suspensionen, jeweils umfassend mindestens eine Spezies von markierten Testbakteriophagen, welche spezifisch an eine nachzuweisende Bakterienart bindet;
B) Zugabe einer Probe, die auf das Vorliegen mindestens einer nachzuweisenden Bakterienart getestet werden soll, zu der einen oder den mehreren Suspensionen;
C) Filtration des resultierenden Reaktionsgemisches, wobei ein Filter mit einer Porengröße von 0,1 µm bis 0,5 µm verwendet wird;
D) Detektion von Bakterien-Bakteriophagen-Komplexen im Retentat, bei Vorliegen mindestens einer nachzuweisenden Bakterienart, wobei die Komplexe aus Bakterien der mindestens einen nachzuweisenden Bakterienart und daran gebundenen Testbakteriophagen der mindestens einen Spezies von Testbakteriophagen bestehen;
E) Detektion von ungebundenen Testbakteriophagen im Filtrat;
F) prozessorgestützte Verarbeitung von erhaltenen Signalen, welche durch die Detektion in den Schritten D) und E) erzeugt werden und Ausgabe von Detektionsergebnissen an einen Benutzer;
wobei die eine oder mehreren Suspensionen ferner einen markierten Referenz-Bakteriophagen umfassen, welcher an keine nachzuweisende Bakterienart bindet und eine von den Testbakteriophagen disjunkte Markierung aufweist, so dass der markierte Referenz-Bakteriophage ebenfalls im Schritt E) detektiert wird; oder wobei, alternativ zu einem markierten Referenz-Bakteriophagen, markierte Mikro- oder Nanopartikel verwendet werden, welche an keine nachzuweisende Bakterienart binden und den Filter aufgrund ihrer geringen Größe passieren können.

2. Verfahren zum Nachweis von Bakterien gemäß Anspruch 1, wobei die nachzuweisenden Bakterienarten ausgewählt werden aus der Gruppe umfassend *Staphylococcus aureus, Methicillin-resistente Staphylococcus aureus (MRSA), Staphylocossus epidermidis, Streptococcus pneumoniae, Streptococcus pyogenes, Haemophilus influenzae, Haemophilus parainfluenzae, Klebsiella pneumoniae, Escherichia coli, Escherichia hermannii, EHEC, Enterobacter cloacae, Enterococcus faecalis, Enterococcus faecum, Pseudomonas aeruginosa, Proteus mirabilis, Proteus vulgaris, Staphylococcus saprophyticus, Bacteroides fragilis, Entercoccus faecium, Enterobacter aerogenes, Serratia marcescens, B-Streptokokken (agalactiae), Chlamydia trachomatis, Chlamydia psittaci, Ureaplasma urealyticum, Mycoplasma hominis, Mycoplasma pneumoniae, Neisseria gonorrhoeae, Neisseria meningitidis, Citrobacter freundii, Moraxella catarrhalis, Stenotrophomonas maltophilia, Pasteurella multocida, Acinetobacter baumannii, Providencia rettgeri, Bordetella pertussis, Bacillus anthracis, Bacillus cereus, Brucella abortus, Brucella melitensis, Clostridium butolinum, Clostridium difficile, Clostridium tetani, Clostridium perfringens, Clamydia trachomatis, Corynebacterium diphtheriae, Francisella tularensis, Gardenella vaginalis, Listeria monocytogenes, Morganella morganii, Mycobacterium leprae, Mycobacterium tuberculosis, Nocardia asteriodes, Salmonella bongori, Salmonella enterica, Shigella spp., Vibrio cholerae, Borrelia burgdorferi, Yersinia pestis, Yersinia enterocolitica, Coxiella burnettii, Aeromonas spp., Plesiomonas spp., Xanthomonas maltophilia, Treponema pallidum, Eikenella corrodens, Spirillum minus, Rickettsia prowazeki, Rickettsia rickettsii, Rickettsia conorii, Cronobacter spp., Campylobacter spp. und Legionella pneumophilia.*

3. Verfahren zum Nachweis von Bakterien gemäß Anspruch 1 oder 2, wobei die eine oder mehreren Suspensionen jeweils zwei oder mehrere Spezies von markierten Testbakteriophagen umfassen, welche jeweils spezifisch an unterschiedliche nachzuweisende Bakterienarten binden und wobei die Markierungen der Spezies disjunkt oder teilweise überlappend sind und wobei die Markierung für die jeweilige Spezies von Testbakteriophagen kennzeichnend ist.

4. Verfahren zum Nachweis von Bakterien gemäß einem der vorhergehenden Ansprüche, wobei die Markierung mindestens ein Reportermolekül mit fluoreszenten oder chemilumineszenten Eigenschaften oder mindestens ein Reportermolekül, welches durch Interaktion mit einem sekundären Molekül Licht emittiert, umfasst.

5. Verfahren zum Nachweis von Bakterien gemäß Anspruch 4, wobei die Markierung jeweils aus mindestens einem fluoreszenten Reportermolekül besteht und die Detektion durch Messung der Fluoreszenz erfolgt, wobei die Anregung mindestens eines fluoreszenten Reportermoleküls im Wellenlängenbereich von 200-1000 nm erfolgt.

6. Verfahren zum Nachweis von Bakterien gemäß Anspruch 4 oder 5, wobei das mindestens eine Reportermolekül an Proteine im Capsid-Bereich der Bakteriophagen gekoppelt ist.

7. Verfahren zum Nachweis von Bakterien gemäß einem der Ansprüche 4 bis 6, wobei die Detektion jeweils durch zeitaufgelöste Messung der Lichtemission erfolgt.

8. Verfahren zum Nachweis von Bakterien gemäß einem der vorhergehenden Ansprüche, ferner umfassend einen Schritt G) des Mischens der Probe mit der einen oder den mehreren Suspensionen und der Inkubation des resultierenden Reaktionsgemisches, wobei Schritt G) auf Schritt B) folgt.

9. Verfahren zum Nachweis von Bakterien gemäß einem der vorhergehenden Ansprüche, ferner umfassend den Schritt einer Vortrennung von Bakterien-Bakteriophagen-Komplexen und ungebundenen Bakteriophagen, wobei die Vortrennung auf Schritt B) oder G) folgt.

10. Reaktionsgefäß zum Nachweis von Bakterien, umfassend
mindestens zwei Kompartimente, welche durch einen Filter voneinander getrennt sind,
wobei der Filter eine Porengröße von 0,1 µm bis 0,5 µm aufweist
wobei ein Kompartiment ein Phagenreservoir ist, welches eine Suspension enthält, die mindestens eine Spezies von markierten Testbakteriophagen umfasst, welche spezifisch an eine nachzuweisende Bakterienart bindet, und
ein Kompartiment ein Auffangreservoir ist, zur Aufnahme des Filtrats nach Zugabe einer Probe zu der Suspension und Filtration durch den Filter;
ein Mittel zur Identifikation des Reaktionsgefäßes; und
mindestens zwei Messfenster,
wobei ein Messfenster im Bereich der Filteroberfläche, welche dem Phagenreservoir zugewandt ist, angeordnet ist, und
ein Messfenster im Bereich des Auffangreservoirs angeordnet ist;
wobei die Suspension ferner einen markierten Referenz-Bakteriophagen umfasst, welcher an keine nachzuweisende Bakterienart bindet und eine von den Testbakteriophagen disjunkte Markierung aufweist; oder wobei das Reaktionsgefäß ein weiteres Kompartiment zwischen Filter und Phagenreservoir umfasst, welches eine Indikatorlösung mit markierten Mikro- oder Nanopartikeln enthält, welche nicht an eine nachzuweisende Bakterienart binden und den Filter aufgrund ihrer geringen Größe passieren können.

11. Reaktionsgefäß gemäß Anspruch 10, wobei die Suspension zwei oder mehrere Spezies von markierten Testbakteriophagen umfasst, welche jeweils spezifisch an unterschiedliche nachzuweisende Bakterienarten binden und wobei die Markierungen der Spezies disjunkt oder teilweise überlappend sind und wobei die Markierung für die jeweilige Spezies von Testbakteriophagen kennzeichnend ist.

12. Reaktionsgefäß gemäß einem der Ansprüche 10 bis 11, ferner umfassend ein Kompartiment zwischen Phagenresevoir und Filter, welches mindestens einen Vorfilter enthält.

13. Kartusche, umfassend
zwei oder mehrere Reaktionsgefäße nach einem der Ansprüche 10-12, welche parallel und/oder seriell zueinander angeordnet sind,
wobei jedes parallele Reaktionsgefäß unterschiedliche Spezies von markierten Testbakteriophagen in der Suspension des Phagenreservoirs enthält, welche jeweils spezifisch an unterschiedliche nachzuweisende Bakterienarten binden.

14. Kartusche nach Anspruch 13, wobei die Markierungen der unterschiedlichen Spezies von markierten Testbakteriophagen disjunkt oder teilweise überlappend sind.

15. Verwendung eines Reaktionsgefäßes gemäß einem der Ansprüche 10 bis 12 oder einer Kartusche gemäß einem der Ansprüche 13 bis 14 zur Durchführung des Verfahrens zum Nachweis von Bakterien gemäß einem der Ansprüche 1 bis 9.

16. Verwendung eines Messgeräts zur Durchführung des Verfahrens zum Nachweis von Bakterien gemäß einem der Ansprüche 1 bis 9, wobei das Messgerät umfasst:
einen Schacht, in den ein Reaktionsgefäß gemäß einem der Ansprüche 10-12 oder eine Kartusche nach Anspruch 13 oder 14 eingeschoben wird;
mindestens zwei Detektions-Optiken, welche jeweils im Bereich der Messfenster des eingeschobenen Reaktionsgefäßes oder der Kartusche angeordnet sind,
wobei die Detektions-Optiken jeweils ein Linsensystem zur Fokussierung von emittiertem Licht der Markierung der Bakteriophagen auf mindestens einen Sensor umfassen, wobei der mindestens eine Sensor das emittierte Licht detektiert;
einen Prozessor, welcher jeweils mit dem mindestens einen Sensor der Detektionsoptiken verbunden ist und welcher die erhaltenen Detektionssignale verarbeitet; und
eine mit dem Prozessor verbundene Ausgabeeinheit, welche die Detektionsergebnissen an einen Benutzer ausgibt.

17. Verwendung eines Messgeräts gemäß Anspruch 16, wobei die Detektions-Optiken ferner jeweils eine Beleuchtungseinheit, welche Licht im Wellenlängenbereich von 200-1000 nm emittiert und ein Linsensystem zur Fokussierung des von der Beleuchtungseinheit emittierten Lichts auf einen Messbereich umfassen, wobei die Beleuchtungseinheit ebenfalls mit dem Prozessor verbunden ist und von diesem gesteuert wird.

18. Verwendung eines Messgeräts gemäß einem der Ansprüche 16 bis 17, wobei das Messgerät ferner ein integriertes Lesegerät umfasst, welches das Mittel zur Identifikation des Reaktionsgefäßes erfasst und die erfassten Daten an den Prozessor weiterleitet, wobei der Prozessor die erfassten Daten verarbeitet und die Ergebnisse der Identifikation über die Ausgabeeinheit an einen Benutzer ausgegeben werden.

## Claims

1. A method for the detection of bacteria comprising the steps of
A) provision of one or more suspensions each comprising at least one species of labelled test bacteriophages which specifically bind to a bacterial species to be detected;
B) addition of a sample to be tested for the presence of at least one bacterial species to be detected to the one or more suspensions;
C) filtration of the resulting reaction mixture, wherein a filter with a pore size of 0.1 µm to 0.5 µm is used;
D) detection of bacteria-bacteriophages-complexes in the retentate, provided that at least one bacterial species to be detected is present, wherein the complexes consist of bacteria of the at least one bacterial species to be detected and test bacteriophages of the at least one species of test bacteriophages bound thereto;
E) detection of unbound test bacteriophages in the filtrate;
F) processor-aided processing of received signals generated by the detection in steps D) and E) and output of detection results to a user,
wherein the one or more suspensions further comprise a labelled reference bacteriophage which does not bind to any bacterial species to be detected and which has a label disjunct from the test bacteriophages such that labelled reference bacteriophage is also detected in step E); or wherein labelled micro- or nanoparticles are used in alternative to a labelled reference bacteriophage, which do not bind to any bacterial species to be detected and can pass through the filter due to their small size.

2. The method for the detection of bacteria according to claim 1, wherein the bacterial species to be detected are selected from the group comprising *Staphylococcus aureus, Methicillin-resistant Staphylococcus aureus (MRSA), Staphylocossus epidermidis, Streptococcus pneumoniae, Streptococcus pyogenes, Haemophilus influenzae, Haemophilus parainfluenzae, Klebsiella pneumoniae, Escherichia coli, Escherichia hermannii, EHEC, Enterobacter cloacae, Enterococcus faecalis, Enterococcus faecum, Pseudomonas aeruginosa, Proteus mirabilis, Proteus vulgaris, Staphylococcus saprophyticus, Bacteroides fragilis, Entercoccus faecium, Enterobacter aerogenes, Serratia marcescens, B-Streptococcus (agalactiae), Chlamydia trachomatis, Chlamydia psittaci, Ureaplasma urealyticum, Mycoplasma hominis, Mycoplasma pneumoniae, Neisseria gonorrhoeae, Neisseria meningitidis, Citrobacter freundii, Moraxella catarrhalis, Stenotrophomonas maltophilia, Pasteurella multocida, Acinetobacter baumannii, Providencia rettgeri, Bordetella pertussis, Bacillus anthracis, Bacillus cereus, Brucella abortus, Brucella melitensis, Clostridium butolinum, Clostridium difficile, Clostridium tetani, Clostridium perfringens, Clamydia trachomatis, Corynebacterium diphtheriae, Francisella tularensis, Gardenella vaginalis, Listeria monocytogenes, Morganella morganii, Mycobacterium leprae, Mycobacterium tuberculosis, Nocardia asteriodes, Salmonella bongori, Salmonella enterica, Shigella spp., Vibrio cholerae, Borrelia burgdorferi, Yersinia pestis, Yersinia enterocolitica, Coxiella burnettii, Aeromonas spp., Plesiomonas spp., Xanthomonas maltophilia, Treponema pallidum, Eikenella corrodens, Spirillum minus, Rickettsia prowazeki, Rickettsia rickettsii, Rickettsia conorii, Cronobacter spp., Campylobacter spp.* and *Legionella pneumophilia.*

3. The method for the detection of bacteria according to claim 1 or 2, wherein the one or more suspensions each comprise two or more species of labeled test bacteriophages which each specifically bind to different bacterial species to be detected and wherein the labels of the species are disjunct or partially overlapping and wherein the label is characteristic of the respective species of test bacteriophages.

4. The method for the detection of bacteria according to any of the preceding claims, wherein the label comprises at least one reporter molecule having fluorescent or chemiluminescent properties or at least one reporter molecule which emits light by interaction with a secondary molecule.

5. The method for the detection of bacteria according to claim 4, wherein the label consists of at least one fluorescent reporter molecule, respectively, and the detection is carried out by measuring the fluorescence, wherein the excitation of at least one fluorescent reporter molecule is carried out in the wavelength range of from 200-1000 nm.

6. The method for the detection of bacteria according to claim 4 or 5, wherein the at least one reporter molecule is coupled to proteins in the capsid region of the bacteriophages.

7. Method for the detection of bacteria according to any one of claims 4 to 6, wherein the detection is carried out by time-resolved measurement of the light emission.

8. The method for the detection of bacteria according to any of the preceding claims, further comprising a step G) of mixing the sample with the one or more suspensions and incubating the resulting reaction mixture, wherein step G) follows step B).

9. The method for the detection of bacteria according to any of the preceding claims, further comprising the step of a pre-separation of bacteria-bacteriophages-complexes and unbound bacteriophages, wherein the pre-separation follows step B) or G).

10. A reaction vessel for the detection of bacteria, comprising
at least two compartments separated from each other by a filter,
wherein the filter has a pore size of 0.1 µm to 0.5 µm
wherein one compartment is a phage reservoir containing a suspension which comprises at least one species of labeled test bacteriophages that specifically bind to a bacterial species to be detected, and
one compartment is a collection reservoir for receiving the filtrate after the addition of a sample to the suspension and filtration through the filter;
a means for identifying the reaction vessel; and
at least two measurement windows,
wherein one measurement window is arranged in the region of the filter surface facing the phage reservoir, and
one measuring window is arranged in the region of the collection reservoir,
wherein the suspension further comprises a labelled reference bacteriophage which does not bind to any bacterial species to be detected and which has a label disjunct from the test bacteriophages; or wherein the reaction vessel comprises a further compartment between the filter and the phage reservoir, which contains an indicator solution with labeled micro- or nanoparticles that do not bind to any bacterial species to be detected and that may pass through the filter due to their small size.

11. The reaction vessel according to claim 10, wherein the suspension comprises two or more species of labeled test bacteriophages which each specifically bind to different bacterial species to be detected and wherein the labels of the species are disjunct or partially overlapping and wherein the label is characteristic of the respective species of test bacteriophages.

12. The reaction vessel according to any one of claims 10 to 11, further comprising a compartment between the phage reservoir and the filter, which contains at least one pre-filter.

13. A cartridge, comprising
two or more reaction vessels according to any one of claims 10-12, which are arranged parallel and/or in series to each other,
wherein each parallel reaction vessel contains different species of labeled test bacteriophages in the suspension of the phage reservoir, each specifically binding to different bacterial species to be detected.

14. The cartridge according to claim 13, wherein the labels of the different species of labeled test bacteriophages are disjunct or partially overlapping.

15. Use of a reaction vessel according to any one of claims 10 to 12 or of a cartridge according to any one of claims 13 to 14 for carrying out the method for the detection of bacteria according to any one of claims 1 to 9.

16. Use of a measuring device for carrying out the method for the detection of bacteria according to any one of claims 1 to 9, wherein said measuring device comprises
a slot into which a reaction vessel according to any one of claims 10-12 or a cartridge according to claim 13 or 14 is inserted;
at least two detection optics, each of which is arranged in the region of the measuring windows of the inserted reaction vessel or cartridge,
wherein the detection optics each comprise a lens system for focusing light emitted by the label of the bacteriophages onto at least one sensor, the at least one sensor detecting the emitted light;
a processor which is connected to the at least one sensor of the detection optics, respectively, and which processes the received detection signals; and
an output unit connected to the processor, which outputs the detection results to a user.

17. Use of a measuring device according to claim 16, wherein the detection optics further each comprise an illumination unit, which emits light in the wavelength range of from 200-1000 nm, and a lens system for focusing the light emitted by the illumination unit onto a measuring area, wherein the illumination unit is also connected to and controlled by the processor.

18. Use of a measuring device according to any one of claims 16 to 17, further comprising an integrated reader that detects the means for identifying the reaction vessel and forwards the detected data to the processor, whereby the processor processes the detected data and outputs the results of the identification to a user via the output unit.

## Revendications

1. Procédé pour la détection de bactéries, comprenant les étapes
A) fourniture d'une ou plusieurs suspensions, chacune comprenant au moins une espèce de bactériophages d'essai marqués, qui se lie spécifiquement à une espèce de bactéries à détecter ;
B) addition d'un échantillon qui doit faire l'objet d'un test portant sur la présence d'au moins une espèce de bactéries à détecter, à la ou aux plusieurs suspensions ;
C) filtration du mélange réactionnel obtenu, par utilisation d'un filtre ayant une taille de pores de 0,1 µm à 0,5 µm ;
D) détection de complexes bactéries-bactériophages dans le rétentat, dans le cas de la présence d'au moins une espèce de bactéries à détecter, les complexes étant constitués de bactéries de l'au moins une espèce de bactéries à détecter et de bactériophages d'essai, qui y sont liés, de l'au moins une espèce de bactériophages d'essai ;
E) détection des bactériophages d'essai non liés dans le filtrat ;
F) traitement assisté par ordinateur des signaux obtenus, qui sont produits par la détection des étapes D) et E), et transmission à un utilisateur des résultats de la détection ;
dans lequel la ou les suspensions comprennent en outre un bactériophage de référence marqué, qui ne se lie à aucune espèce de bactéries à détecter et présente un marquage disjoint des bactériophages d'essai, de telle sorte que le bactériophage de référence marqué soit lui aussi détecté dans l'étape E) ; ou dans lequel, à la place d'un bactériophage de référence marqué, on utilise des micro- ou des nanoparticules marquées, qui ne se lient à aucune espèce de bactéries à détecter et qui peuvent passer à travers le filtre du fait de leur faible taille.

2. Procédé pour la détection de bactéries selon la revendication 1, dans lequel les espèces de bactéries à détecter sont choisies dans le groupe comprenant *Staphylococcus aureus, Staphylococcus aureus* résistant à la méthicilline *(SARM), Staphylocossus epidermidis, Streptococcus pneumoniae, Streptococcus pyogenes, Haemophilus influenzae, Haemophilus parainfluenzae, Klebsiella pneumoniae, Escherichia coli, Escherichia hermannii, EHEC, Enterobacter cloacae, Enterococcus faecalis, Enterococcus faecum, Pseudomonas aeruginosa, Proteus mirabilis, Proteus vulgaris, Staphylococcus saprophyticus, Bacteroides fragilis, Enterococcus faecium, Enterobacter aerogenes, Serratia marcescens, streptocoques B (agalactiae), Chlamydia trachomatis, Chlamydia psittaci, Ureaplasma urealyticum, Mycoplasma hominis, Mycoplasma pneumoniae, Neisseria gonorrhoeae, Neisseria meningitidis, Citrobacter freundii, Moraxella catarrhalis, Stenotrophomonas maltophilia, Pasteurella multocida, Acinetobacter baumannii, Providencia rettgeri, Bordetella pertussis, Bacillus anthracis, Bacillus cereus, Brucella abortus, Brucella melitensis, Clostricium butolinum, Clostridium difficile, Clostridium tetani, Clostridium perfringens, Clamydia trachomatis, Corynebacterium diphtheriae, Francisella tularensis, Gardenella vaginalis, Listeria monocytogenes, Morganella morganii, Mycobacterium leprae, Mycobacterium tuberculosis, Nocardia asteroides, Salmonella bongori, Salmonella enterica, Shigella spp., Vibrio cholerae, Borrelia burgdorferi, Yersinia pestis, Yersinia enterocolitica, Coxiella burnettii, Aeromonas spp., Plesiomonas spp., Xanthomonas maltophilia, Treponema pallidum, Eikenella corrodens, Spirillum minus, Rickettsia prowazeki, Rickettsia rickettsii, Rickettsia conorii, Cronobacter spp., Camphylobacter spp.* et *Legionella pneumophilia.*

3. Procédé pour la détection de bactéries selon la revendication 1 ou 2, dans lequel la ou les suspensions comprennent chacune deux ou plusieurs espèces de bactériophages d'essai marqués, dont chacun se lie spécifiquement à des espèces de bactéries à détecter différentes, et dans lequel les marquages des espèces se chevauchent d'une manière disjointe ou partielle, et dans lequel le marquage est caractéristique de l'espèce considérée de bactériophages d'essai.

4. Procédé pour la détection de bactéries selon l'une des revendications précédentes, dans lequel le marquage comprend au moins une molécule rapporteuse présentant des propriétés fluorescentes ou chimioluminescentes ou au moins une molécule rapporteuse qui émet une lumière par interaction avec une molécule secondaire.

5. Procédé pour la détection de bactéries selon la revendication 4, dans lequel chaque marquage est constitué d'au moins une molécule rapporteuse fluorescente, et la détection a lieu par mesure de la fluorescence, l'excitation d'au moins une molécule rapporteuse fluorescente s'effectuant dans la plage de longueurs d'onde de 200 à 1000 nm.

6. Procédé pour la détection de bactéries selon la revendication 4 ou 5, dans lequel l'au moins une molécule rapporteuse est couplée à des protéines dans le domaine de la capside des bactériophages.

7. Procédé pour la détection de bactéries selon l'une des revendications 4 à 6, dans lequel chaque détection est effectuée par une mesure avec résolution dans le temps de l'émission de lumière.

8. Procédé pour la détection de bactéries selon l'une des revendications précédentes, comprenant en outre une étape G) de mélange de l'échantillon avec la ou les suspensions, et l'incubation du mélange réactionnel obtenu, l'étape G) suivant l'étape B).

9. Procédé pour la détection de bactéries selon l'une des revendications précédentes, comprenant en outre l'étape d'une séparation préalable de complexes bactéries-bactériophages et de bactériophages non liés, la séparation préalable suivant l'étape B) ou l'étape G).

10. Récipient de réaction pour la détection de bactéries, comprenant
au moins deux compartiments, qui sont séparés l'un de l'autre par un filtre,
le filtre présentant une taille de pores de 0,1 µm à 0,5 µm,
dans lequel un compartiment est un réservoir de phages, qui contient une suspension qui comprend au moins une espèce de bactériophages d'essai marqués, qui se lie spécifiquement à une espèce de bactéries à détecter, et
un compartiment est un réservoir récepteur, destiné à recevoir le filtrat après addition d'un échantillon à la suspension et filtration à travers le filtre ;
un moyen pour l'identification du récipient de réaction ; et
au moins deux fenêtres de mesure,
au moins une fenêtre étant disposée dans la zone de la surface du filtre qui est dirigée vers le réservoir de phages, et
une fenêtre de mesure est disposée dans la zone du réservoir récepteur ;
dans lequel la suspension comprend en outre un bactériophage de référence marqué, qui ne se lie à aucune espèce de bactéries à détecter et présente un marquage disjoint des bactériophages d'essai, ou dans lequel le récipient de réaction comprend un compartiment supplémentaire entre le filtre et le réservoir de phages, qui contient une solution indicatrice avec des micro- ou des nanoparticules marquées, qui ne se lient pas à une espèce de bactéries à détecter et peuvent passer à travers le filtre du fait de leur faible taille.

11. Récipient de réaction selon la revendication 10, dans lequel la suspension comprend deux ou plusieurs espèces de bactériophages d'essai marqués, dont chacune se lie spécifiquement à des espèces de bactéries à détecter différentes, et dans lequel les marquages des espèces se chevauchent d'une manière disjointe ou partielle, et dans lequel le marquage est caractéristique de l'espèce considérée de bactériophages d'essai.

12. Récipient de réaction selon l'une des revendications 10 à 11, comprenant en outre un compartiment entre le réservoir de phages et le filtre, qui contient au moins un préfiltre.

13. Cartouche, comprenant
deux ou plusieurs récipients de réaction selon l'une des revendications 10 à 12, qui sont disposés parallèlement les uns aux autres et/ou en série les uns par rapport aux autres,
dans laquelle chaque récipient de réaction disposés en parallèle contient des espèces différentes de bactériophages d'essai marqués dans la suspension du réservoir de phages, dont chacune se lie spécifiquement à des espèces de bactéries à détecter différentes.

14. Cartouche selon la revendication 13, dans laquelle les marquages des espèces différentes de bactériophages d'essai marqués se chevauchent d'une manière disjointe ou partielle.

15. Utilisation d'un récipient de réaction selon l'une des revendications 10 à 12 ou d'une cartouche selon l'une des revendications 13 à 14 pour la mise en œuvre du procédé pour la détection de bactéries selon l'une des revendications 1 à 9.

16. Utilisation d'un appareil de mesure pour la mise en œuvre du procédé pour la détection des bactéries selon l'une des revendications 1 à 9, l'appareil de mesure comprenant :
un puits dans lequel un récipient de réaction selon l'une des revendications 10 à 12 ou une cartouche selon la revendication 13 ou 14 est inséré ;
au moins deux optiques de détection, dont chacune est disposée dans la zone des fenêtres de mesure du récipient de réaction inséré ou de la cartouche,
les optiques de détection comprenant chacune un système de lentilles pour la focalisation de la lumière émise par le marquage des bactériophages sur au moins un capteur, l'au moins un capteur détectant la lumière émise ;
un processeur, qui est relié à chacun du ou desdits au moins un capteur des optiques de détection, et qui traite les signaux de détection obtenus ; et
une unité de transmission reliée au processeur, qui transmet à un utilisateur les résultats de la détection.

17. Utilisation d'un appareil de mesure selon la revendication 16, dans lequel chacune des optiques de détection comprend en outre une unité d'éclairage, qui émet une lumière dans la plage de longueurs d'onde de 200 à 1000 nm, et un système de lentilles pour la focalisation de la lumière émise par l'unité d'éclairage sur une zone de mesure, l'unité d'éclairage étant elle aussi reliée au processeur et étant commandée par ce dernier.

18. Utilisation d'un appareil de mesure selon l'une des revendications 16 à 17, l'appareil de mesure comprenant en outre un appareil de lecture intégré, qui saisit le moyen pour l'identification du récipient de réaction et transfère au processeur les données saisies, le processeur traitant les données saisies, et les résultats de l'identification étant transmis à un utilisateur par l'intermédiaire de l'unité de transmission.
